# EUROPEAN PATENT APPLICATION

(11) **EP 4 759 253 A1**
(43) Date of publication of application: **17.06.2026**
(21) Application number: 23948263.1
(22) Date of filing: 10.11.2023
(51) Int. Cl.: A61B 18/00, A61B 18/12, A61B 18/14, A61C 19/06

(54) **PULSE ABLATION DEVICE AND ABLATION DEVICE FOR ORAL CAVITY**

(30) Priority: 08.08.2023 CN 202310995761; 08.08.2023 CN 202310997795
(71) Applicant: Merryspring Medical Technology (Zhejiang) Co, Ltd., Jiaxing, Zhejiang 314000 (CN)
(72) Inventor: XU, Shuhan, Jiaxing, Zhejiang 314000 (CN); TAO, Lin, Jiaxing, Zhejiang 314000 (CN); YANG, Yong, Jiaxing, Zhejiang 314000 (CN); HOU, Lin, Jiaxing, Zhejiang 314000 (CN); HUANG, Congtao, Jiaxing, Zhejiang 314000 (CN); TANG, Zhongchao, Jiaxing, Zhejiang 314000 (CN); FU, Jiawei, Jiaxing, Zhejiang 314000 (CN); YE, Ping, Jiaxing, Zhejiang 314000 (CN)
(74) Representative: De Arpe Tejero, Manuel
(86) International application number: PCT/CN2023/130984
(87) International publication number: WO 2025/030700

(57) **Abstract**

Provided in the present application are a pulse ablation device and an ablation device for the oral cavity. The pulse ablation device is applied to lesion tissue in the oral cavity and comprises an outer electrode, an inner electrode, an electrode fixing device and an operating bar. The outer electrode is fixedly connected to the electrode fixing device, the inner electrode is fixedly connected to the electrode fixing device, and the electrode fixing device is fixedly connected to the operating bar. The outer electrode and the inner electrode are both provided at the distal end of the electrode fixing device. The outer electrode surrounds the outer circumference of the inner electrode; a discharging gap is formed between the outer electrode and the inner electrode; and the discharging gap can be broken down by an electric current to form pulses. The pulse ablation device provided by the present application uses the outer electrode and the inner electrode to form an electrode pair, and can accurately emit pulses to lesion tissue in the oral cavity, so as to perform pulse ablation treatment on the lesion tissue in the oral cavity. The pulse ablation device has a simple structure and achieves a good treatment effect.

## Description

### TECHNICAL FIELD

This application relates to the field of oral ablation technology, particularly to a pulse ablation device and an ablation device for an oral cavity.

### BACKGROUND

The treatment of oral diseases often employs ablation as a therapeutic approach. Ablation surgery, a minimally invasive procedure, can be used to treat both benign and malignant tumors, primarily by inducing denaturation or coagulative necrosis of tumor cells through high temperatures, thereby achieving tumor eradication.

Currently, radiofrequency ablation and microwave ablation are widely used in clinical practice. However, these ablation methods often result in extensive tissue necrosis, leading to issues such as restricted mouth opening and suboptimal treatment outcomes.

Oral mucosal diseases encompass all conditions within the oral cavity, excluding dental diseases. Common examples include lichen planus, oral ulcers, Behçet's disease, tongue diseases, lip diseases, dry mouth, halitosis, gingivitis, and pharyngitis, among others.

In the prior art, treatment modalities for oral mucosal diseases typically include radiofrequency ablation, microwave ablation, laser ablation, or ultrasonic ablation. All these techniques fall under the category of thermal ablation, which relies on high temperatures to denature proteins and induce cell death. However, this thermal ablation approach lacks selectivity in treating oral mucosal tissues and can cause extensive thermal damage to the oral cavity.

Therefore, there is an urgent need for a pulse ablation device and an oral ablation device to address the aforementioned technical challenges.

### SUMMARY

This application provides a pulse ablation device. Composed of electrode pairs formed by an outer electrode and an inner electrode, it can precisely emit pulses to oral lesion tissues for pulse ablation treatment, featuring a simple structure and good therapeutic effects.

This application provides a pulse ablation device for application to oral lesion tissues, which comprises an outer electrode, an inner electrode, an electrode fixing device, and an operating rod.

The outer electrode is fixedly connected to the electrode fixing device, and the inner electrode is also fixedly connected to the electrode fixing device; the electrode fixing device is fixedly connected to the operating rod.

Both the outer electrode and the inner electrode are arranged at the distal end of the electrode fixing device.

The outer electrode is arranged around the outer peripheral side of the inner electrode, and a discharge gap is formed between the outer electrode and the inner electrode, which can be broken down by electric current to generate pulses.

Further, the electrode fixing device comprises an electrode fixing base and a base rubber-coating part. The electrode fixing base is fixedly connected to the operating rod.

The proximal ends of both the outer electrode and the inner electrode are fixedly connected to the distal end of the electrode fixing base.

The base rubber-coating part is wrapped around the distal end of the electrode fixing base and is used to fix the outer electrode and the inner electrode onto the electrode fixing base.

Further, both the outer electrode and the inner electrode protrude from the distal side of the base rubber-coating part.

Further, the protrusion height of the outer electrode from the distal side of the base rubber-coating part is 0.1 - 0.3 mm, and the protrusion height of the inner electrode from the distal side of the base rubber-coating part is 0.1 - 0.3 mm.

Further, the operating rod comprises a first sub-housing and a second sub-housing that are fixedly connected.

The proximal end of the electrode fixing base is fixedly arranged between the first sub-housing and the second sub-housing.

The distal end of at least one of the first sub-housing and the second sub-housing is fixedly connected to the electrode fixing base and extends into the base rubber-coating part.

Further, an outer electrode positioning structure is provided on the distal side of the electrode fixing base, and the outer electrode is fixedly connected to the outer electrode positioning structure.

The outer electrode positioning structure has an outer electrode abutment surface, and at least one first recessed groove is provided on the outer electrode abutment surface.

Further, an inner electrode positioning structure is provided on the distal side of the electrode fixing base, and the inner electrode is fixedly connected to the inner electrode positioning structure.

The inner electrode positioning structure has an inner electrode abutment surface, and at least one second recessed groove is provided on the inner electrode abutment surface.

Further, the outer electrode positioning structure is arranged on the outer peripheral side of the inner electrode positioning structure, and the outer electrode positioning structure is spaced apart from the inner electrode positioning structure, with a first gap between them.

At least one through-hole is provided on the outer electrode positioning structure, and the through-hole is in communication with the first gap.

Further, the pulse ablation device also comprises an outer electrode connecting wire and an inner electrode connecting wire.

The outer electrode connecting wire is fixedly connected to the outer electrode, and the inner electrode connecting wire is fixedly connected to the inner electrode.

At least one wire retaining groove is provided on the electrode fixing base, and the outer electrode connecting wire and the inner electrode connecting wire are respectively engaged with the wire retaining groove.

Further, a first wire routing hole is provided on the electrode fixing base, a second wire routing hole is provided inside the operating rod, the first wire routing hole is in communication with the second wire routing hole, and the wire retaining groove is arranged on the first wire routing hole.

Both the first wire routing hole and the second wire routing hole are used to accommodate the outer electrode connecting wire and the inner electrode connecting wire.

Further, the electrode fixing device has a curved section, and the angle between the curved section of the electrode fixing device and the axial direction of the operating rod is 35 - 50°.

Further, the operating rod comprises multiple operating sections, and each operating section includes a curved section. The curved sections of adjacent operating sections bend in different directions.

The curved section of the operating section closest to the electrode fixing device among the multiple operating sections bends in a different direction from the curved section of the electrode fixing device.

Further, the multiple operating sections include a first operation zone and a second operation zone, and the first operation zone is arranged close to the electrode fixing device.

The second operation zone, the first operation zone, and the electrode fixing device are sequentially connected in an S-shape.

Further, the pulse ablation device meets at least one of the following characteristics.

The angle between the first operation zone and the axial direction of the operating rod is 15 - 25°.

The angle between the second operation zone and the axial direction of the operating rod is 15 - 25°.

Further, the outer electrode is ring-shaped, and the inner electrode is circular.

The outer electrode and the inner electrode are concentrically arranged.

Further, the operating rod is a variable-diameter operating rod, and along the direction from the proximal end to the distal end of the operating rod, the radial dimension of the variable-diameter operating rod decreases.

On the other hand, this application also provides an oral ablation device. By using at least one first microneedle and at least one second microneedle arranged at the end side of the operating handle to emit pulses to target tissues, it can selectively treat oral lesion tissues and improve the therapeutic effect of oral ablation.

The oral ablation device comprises a first electrode, a second electrode, and an operating handle.

The first electrode is fixedly connected to the operating handle, and the second electrode is fixedly connected to the operating handle. The first electrode and the second electrode are arranged opposite to each other.

The first electrode has at least one first microneedle, and the second electrode has at least one second microneedle. Both the first microneedles and the second microneedles protrude from the end side of the operating handle.

The first electrode and the second electrode are used to emit pulses to target tissues through the first microneedles and the second microneedles.

Further, the operating handle comprises a support housing and an electrode adapter.

The electrode adapter is fixedly connected to the end side of the support housing.

One end of the first electrode, away from the tip of the first microneedle, and one end of the second electrode, away from the tip of the second microneedle, are respectively fixedly connected to the end of the electrode adapter away from the support housing. The first electrode and the second electrode are fixed on the opposite end sides of the end of the electrode adapter away from the support housing.

Further, the operating handle also comprises an electrode fixing part.

The electrode fixing part is sleeved on the electrode adapter and covers the connecting portion, between the first electrode and the electrode adapter, and the connecting portion, between the second electrode and the electrode adapter. The electrode fixing part is configured to fix the first electrode and the second electrode respectively to the end side of the electrode adapter.

The first microneedles and the second microneedles both protrude outward from the end side of the electrode fixing part.

Further, the support housing comprises a first shell and a second housing. After the first shell and the second housing are fixedly connected, an inner cavity of the shell is formed.

One end of the electrode adapter extends into the inner cavity of the shell and is fixed between the first shell and the second housing.

At least one of the first shell and the second housingl extends into the electrode fixing part and is fixedly connected to the electrode adapter.

Further, the end of the electrode adapter has a first electrode positioning portion and a second electrode positioning portion.

The first electrode has a third electrode positioning portion that is compatible with the first electrode positioning portion. The first electrode positioning portion and the third electrode positioning portion can abut against each other to restrict the movement of the first electrode.

The second electrode has a fourth electrode positioning portion that is compatible with the second electrode positioning portion. The second electrode positioning portion and the fourth electrode positioning portion can abut against each other to restrict the movement of the second electrode.

Further, a groove is provided at the end of the electrode adapter, and the electrode fixing part can extend into the groove.

Further, the end of the electrode adapter also has a first electrode assembly pin and a second electrode assembly pin.

The first electrode has a first electrode assembly hole that is compatible with the first electrode assembly pin, and the first electrode assembly pin can protrude through the first electrode assembly hole.

The second electrode has a second electrode assembly hole that is compatible with the second electrode assembly pin, and the second electrode assembly pin can protrude through the second electrode assembly hole.

Further, the first electrode comprises multiple first microneedles and a first electrode connecting portion that is fixedly connected to the multiple first microneedles. The multiple first microneedles are arranged adjacent to each other at the end side of the first electrode connecting portion, and a first arc-shaped connecting structure is formed between adjacent first microneedles.

The second electrode comprises multiple second microneedles and a second electrode connecting portion that is fixedly connected to the multiple second microneedles. The multiple second microneedles are arranged adjacent to each other at the end side of the second electrode connecting portion, and a second arc-shaped connecting structure is formed between adjacent second microneedles.

Further, the cross-sectional dimension of the distal end of the first microneedle is smaller than that of the proximal end of the first microneedle.

The cross-sectional dimension of the distal end of the second microneedle is smaller than that of the proximal end of the second microneedle.

Further, the first microneedle comprises a first conical end, which is arranged at the distal end of the first microneedle.

The second microneedle comprises a second conical end, which is arranged at the distal end of the second microneedle.

Further, the operating handle comprises multiple operating sections connected in sequence. Each operating section includes an arc-shaped section, and the arc-shaped sections of adjacent operating sections bend in different directions.

Further, the multiple operating sections include a first gripping section, a second gripping section, and an insertion section.

The first gripping section, the second gripping section, and the insertion section are connected in sequence in an S-shape, and the insertion section is connected to the first electrode and the second electrode respectively.

Further, the oral ablation device has an axial direction of the operating handle, and the oral ablation device meets at least one of the following characteristics:
The first gripping section has a first bending angle with respect to the axial direction of the operating handle, and the first bending angle is 10 - 30°.

The second gripping section has a second bending angle with respect to the axial direction of the operating handle, and the second bending angle is 10 - 30°.

The insertion section has a third bending angle with respect to the axial direction of the operating handle, and the third bending angle is 30 - 55°.

Further, the oral ablation device also comprises a first wire and a second wire.

The first electrode is provided with a first wire connection hole, which is used for the first wire to pass through and be fixedly connected to the first wire.

The second electrode is provided with a second wire connection hole, which is used for the second wire to pass through and be fixedly connected to the second wire.

Further, the length by which the first microneedle protrudes from the end side of the operating handle is 1 - 3 mm, and the length by which the second microneedle protrudes from the end side of the operating handle is 1 - 3 mm.

Further, the operating handle is a variable-diameter operating handle, and the radial dimension of the variable-diameter operating handle decreases from the proximal end to the distal end.

Further, the shape of the first electrode is sheet-like, and the shape of the second electrode is sheet-like.

Implementing the embodiments of this application has the following beneficial effects.
1.The pulse ablation device of this application, applied to oral lesion tissues, comprises an outer electrode, an inner electrode, an electrode fixing device, and an operating rod. The outer electrode is fixedly connected to the electrode fixing device, and the inner electrode is fixedly connected to the electrode fixing device. The electrode fixing device is fixedly connected to the operating rod. Both the outer electrode and the inner electrode are arranged at the distal end of the electrode fixing device. The outer electrode is arranged around the outer peripheral side of the inner electrode, and a discharge gap is formed between the outer electrode and the inner electrode. This discharge gap can be broken down by current to form pulses. The pulse ablation device provided in this application forms an electrode pair through the separately arranged outer electrode and inner electrode, which can precisely emit pulses to oral lesion tissues for pulse ablation treatment of oral lesion tissues. It has a simple structure and good therapeutic effect.
2.The oral ablation device of this application comprises a first electrode, a second electrode, and an operating handle. The first electrode is fixedly connected to the operating handle, and the second electrode is fixedly connected to the operating handle. The first electrode and the second electrode are arranged opposite to each other. The first electrode has at least one first microneedle, and the second electrode has at least one second microneedle. Both the first microneedles and the second microneedles protrude from the end side of the operating handle. The first electrode and the second electrode are used to emit pulses to target tissues through the first microneedles and the second microneedles. The oral ablation device of this application can selectively treat oral lesion tissues by emitting pulses to target tissues through at least one first microneedle and at least one second microneedle arranged at the end side of the operating handle, thereby improving the therapeutic effect of oral ablation.

### BRIEF DESCRIPTION OF THE DRAWINGS

To more clearly illustrate the technical solutions in the embodiments of this application, a brief introduction will be given below to the accompanying drawings required for the description of the embodiments. It is evident that the accompanying drawings described below merely represent some embodiments of this application. For those of ordinary skill in the field, other accompanying drawings can also be obtained based on these without the need for creative effort.
FIG. 1 is a partial structural schematic diagram of a pulse ablation device provided in Embodiments 1 to 3 of this application;
FIG. 2 is a partial structural schematic diagram of a pulse ablation device provided in Embodiments 1 to 3 of this application;
FIG. 3 is a partial structural schematic diagram of a pulse ablation device provided in Embodiments 1 to 3 of this application;
FIG. 4 is a partial structural schematic diagram of a pulse ablation device provided in Embodiments 1 to 3 of this application;
FIG. 5 is a partial structural schematic diagram of a pulse ablation device provided in Embodiments 1 to 3 of this application;
FIG. 6 is a partial structural schematic diagram of a pulse ablation device provided in Embodiments 1 to 3 of this application;
FIG. 7 is a partial structural schematic diagram of a pulse ablation device provided in Embodiments 1 to 3 of this application;
FIG. 8 is a structural schematic diagram of a pulse ablation device provided in Embodiments 1 to 3 of this application;
FIG. 9 is a structural schematic diagram of a pulse ablation device provided in Embodiments 1 to 3 of this application;
FIG. 10 is a partial structural schematic diagram of an oral ablation device provided in Embodiments 4 to 6 of this application;
FIG. 11 is an exploded schematic diagram of an oral ablation device provided in Embodiments 4 to 6 of this application;
FIG. 12 is a partial structural schematic diagram of an oral ablation device provided in Embodiments 4 to 6 of this application;
FIG. 13 is a partial structural schematic diagram of an electrode adapter provided in Embodiments 4 to 6 of this application;
FIG. 14 is a structural schematic diagram of a first electrode provided in Embodiments 4 to 6 of this application;
FIG. 15 is a structural schematic diagram of a second electrode provided in Embodiments 4 to 6 of this application;
FIG. 16 is a structural schematic diagram of an oral ablation device provided in Embodiments 4 to 6 of this application;
FIG. 17 is a structural schematic diagram of an oral ablation device provided in Embodiments 4 to 6 of this application;
FIG. 18 is a structural schematic diagram of an oral ablation device provided in Embodiments 4 to 6 of this application.

The following is a supplementary description of the accompanying drawings:
10 - Outer electrode; 20 - Inner electrode; 30 - Electrode fixing device; 31 - Electrode fixing base; 311 - Outer electrode positioning structure; 3111 - Outer electrode abutment surface; 3112 - First recessed groove; 3113 - Outer electrode positioning surface; 3121 - Inner electrode abutment surface; 3122 - Second recessed groove; 3123 - Inner electrode positioning surface; 312 - Inner electrode positioning structure; 313 - First gap; 314 - Through-hole; 315 - Wire retaining slot; 316 - First wire routing slot hole; 32 - rubber-coated part; 40 - Operating rod; 41 - First sub-housing; 42 - Second sub-housing; 43 - Second wire routing slot hole; 44 - First operation zone; 45 - Second operation zone; 50 - First electrode; 51 - First microneedle; 52 - Third electrode positioning portion; 53 - First electrode assembly hole; 54 - First electrode connecting portion; 55 - First wire connection hole; 60 - Second electrode; 61 - Second microneedle; 62 - Fourth electrode positioning portion; 63 - Second electrode assembly hole; 64 - Second electrode connecting portion; 65 - Second wire connection hole; 70 - Operating handle; 71 - Support housing; 711 - First housing; 712 - Second housing; 72 - Electrode adapter; 721 - First electrode positioning portion; 722 - Second electrode positioning portion; 723 - Groove; 724 - First electrode assembly pin; 725 - Second electrode assembly pin; 73 - Electrode fixing part; 74 - First gripping area; 75 - Second gripping area; 76 - Insertion area.

### DETAILED DESCRIPTION

To enable those skilled in the art to better understand the technical solutions in this application, the technical solutions in the embodiments of this application will be described clearly and completely in conjunction with the accompanying drawings in the embodiments of this application. It is evident that the described embodiments are merely a portion, rather than all, of the embodiments of this utility model. All other embodiments obtained by ordinary technicians in this field without making creative efforts based on the embodiments in this application fall within the scope of protection of this application.

It should be noted that the terms "first," "second," etc., in the description, claims, and the above-mentioned accompanying drawings of this application are used to distinguish similar objects and are not necessarily used to describe a specific order or sequence. It should be understood that the data used in this way can be interchanged as appropriate, so that the embodiments of this application described herein can be implemented in sequences other than those illustrated or described herein. In addition, the terms "include" and "have," as well as any variations thereof, are intended to cover non-exclusive inclusions. For example, a process, method, device, product, or equipment that includes a series of steps or units is not necessarily limited to those steps or units clearly listed, but may include other steps or units that are not clearly listed or are inherent to such processes, methods, products, or equipment.

### Embodiment 1

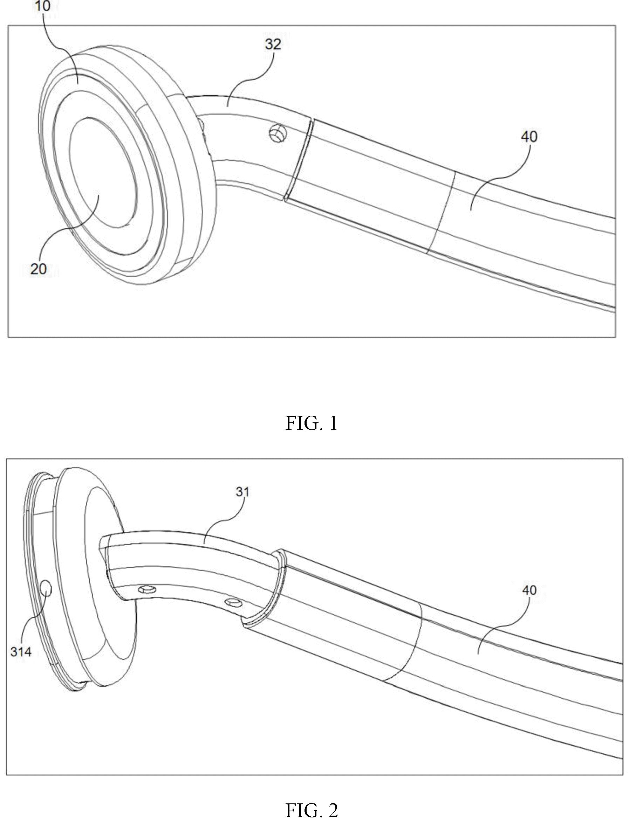

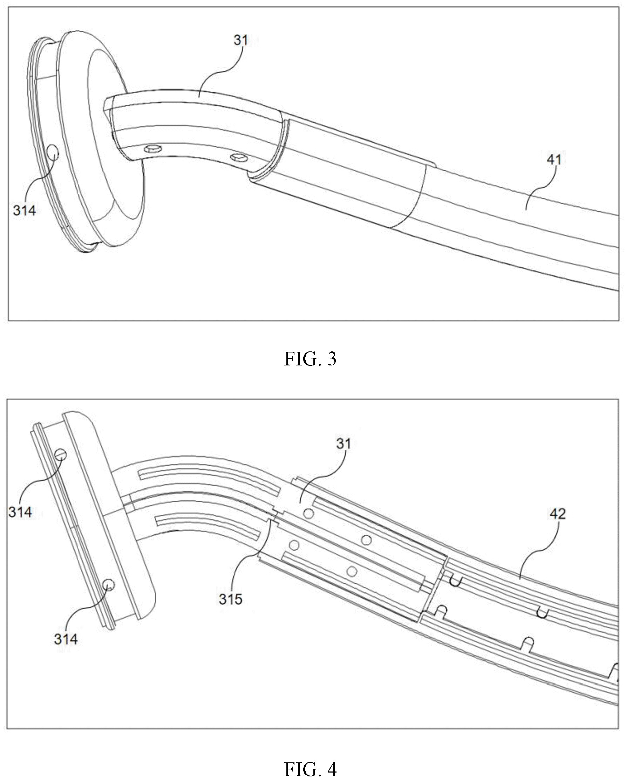

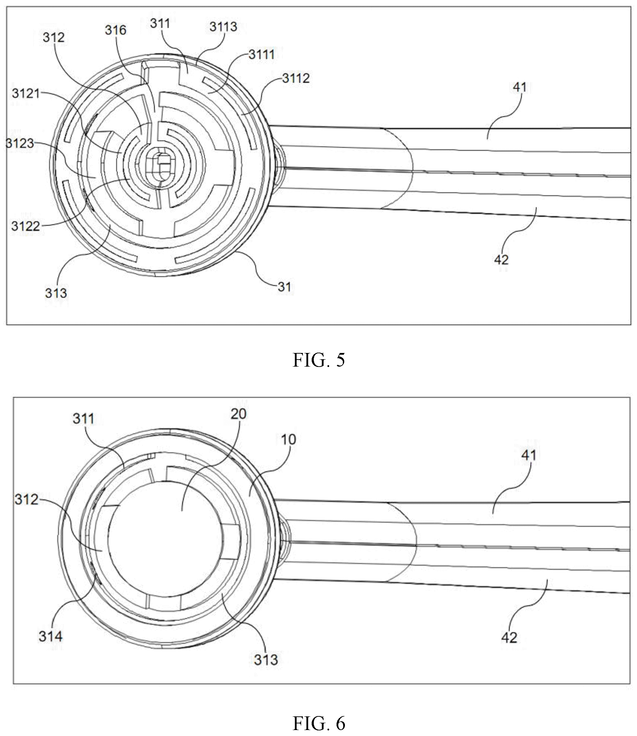

The technical solutions in the embodiments of this application are introduced below in conjunction with FIGS. 1-9. The accompanying drawings do not impose any limiting effect on the content of the application as described in the claims.

This specification provides method operation steps as shown in the embodiments or flowcharts, but based on routine or non-creative labor, more or fewer operation steps may be included. The sequence of steps listed in the embodiments is merely one way among many possible execution sequences and does not represent the only execution sequence.

Referring to FIGS. 1-9, an embodiment of this application provides a pulse ablation device for application to oral lesion tissues, comprising an outer electrode 10, an inner electrode 20, an electrode fixing device 30, and an operating rod 40. The outer electrode 10 is fixedly connected to the electrode fixing device 30, and the inner electrode 20 is fixedly connected to the electrode fixing device 30. The electrode fixing device 30 is fixedly connected to the operating rod 40. Both the outer electrode 10 and the inner electrode 20 are arranged at the distal end of the electrode fixing device 30. The outer electrode 10 is arranged around the outer peripheral side of the inner electrode 20, and a discharge gap is formed between the outer electrode 10 and the inner electrode 20. This discharge gap can be broken down by current to form pulses.

In some embodiments, the outer electrode 10 and the inner electrode 20 are respectively fixedly connected to the distal end of the electrode fixing device 30, and the electrode fixing device 30 is used to fix the relative positions of the outer electrode 10 and the inner electrode 20.

Specifically, the outer electrode 10 and the inner electrode 20 form an electrode pair that emits pulses to oral lesion tissues for pulse ablation treatment of the oral lesion tissues.

In some embodiments, the distal end of the outer electrode 10 has a first tissue-contacting functional surface, which is used to contact oral lesion tissues. Correspondingly, the distal end of the inner electrode 20 has a second tissue-contacting functional surface, which is also used to contact oral lesion tissues.

Specifically, the outer electrode 10 and the inner electrode 20 can form an electrode pair that emits pulses with a microsecond-level width to oral lesion tissues. This causes the stability of the cell membrane surfaces of the oral lesion tissues to be compromised, resulting in the appearance of multiple hydrophilic micropores on the cell surfaces of the oral lesion tissues. This disrupts the homeostasis of the oral lesion tissue cells, leading to their death, thereby achieving the therapeutic effect of pulse ablation on oral lesion tissues.

It can be understood that different oral tissue cells have different damage thresholds. By controlling the magnitude of the pulse energy emitted by the outer electrode 10 and the inner electrode 20, the pulses emitted by the outer electrode 10 and the inner electrode 20 can be precisely targeted to the lesion site, making pulse ablation treatment tissue-cell selective. By controlling the magnitude of the pulse energy emitted by the outer electrode 10 and the inner electrode 20 to correspond to the oral lesion tissue cells, the pulses will only target the oral lesion tissue cells for pulse ablation treatment without damaging other tissue cells.

The pulse ablation device of this application, applied to oral lesion tissues, comprises an outer electrode, an inner electrode, an electrode fixing device, and an operating rod. The outer electrode is fixedly connected to the electrode fixing device, and the inner electrode is fixedly connected to the electrode fixing device. The electrode fixing device is fixedly connected to the operating rod. Both the outer electrode and the inner electrode are arranged at the distal end of the electrode fixing device. The outer electrode is arranged around the outer peripheral side of the inner electrode, and a discharge gap is formed between the outer electrode and the inner electrode. This discharge gap can be broken down by current to form pulses. The pulse ablation device provided in this application forms an electrode pair through the separately arranged outer electrode and inner electrode, enabling precise emission of pulses to oral lesion tissues for pulse ablation treatment of oral lesion tissues. It has a simple structure and good therapeutic effect.

In the embodiments of this application, the electrode fixing device 30 comprises an electrode fixing base 31 and a rubber-coated part 32. The electrode fixing base 31 is fixedly connected to the operating rod 40. The proximal ends of both the outer electrode 10 and the inner electrode 20 are fixedly connected to the distal end of the electrode fixing base 31. The rubber-coated part 32 is wrapped around the distal end of the electrode fixing base 31 and is used to secure the outer electrode 10 and the inner electrode 20 onto the electrode fixing base 31.

Specifically, the electrode fixing base 31 is fixedly connected to the distal end of the operating rod 40.

Specifically, the electrode fixing base 31 is engaged with the operating rod 40 through a pin-in-hole fit, and the electrode fixing base 31 and the operating rod 40 are fixedly connected by laser welding.

Specifically, the proximal end of the outer electrode 10 is fixedly connected to the distal end of the electrode fixing base 31. Correspondingly, the proximal end of the inner electrode 20 is also fixedly connected to the distal end of the electrode fixing base 31.

In some embodiments, the rubber-coated part 32 is sleeved over the electrode fixing base 31 and covers the connection portions between the outer electrode 10 and the electrode fixing base 31, as well as between the inner electrode 20 and the electrode fixing base 31. The rubber-coated part 32 is used to secure the outer electrode 10 and the inner electrode 20 respectively to the end side of the electrode fixing base 31. Both the outer electrode 10 and the inner electrode 20 extend outward from the end side of the rubber-coated part 32.

Specifically, the electrode fixing base 31 and the rubber-coated part 32 cooperate to fix the outer electrode 10 and the inner electrode 20.

In some embodiments, the rubber-coated part 32 can be a soft rubber coating that is wrapped and fixed onto the electrode fixing base 31 through injection molding to secure the outer electrode 10 and the inner electrode 20.

In the embodiments of this application, configuring the rubber-coated part 32 as a soft rubber coating wrapped around the end side of the electrode fixing base 31 can enhance the fixing effect on the outer electrode 10 and the inner electrode 20, preventing the detachment of the outer electrode 10 and/or the inner electrode 20 during the pulse ablation process, and improving the operational safety of the pulse ablation device.

In the embodiments of this application, both the outer electrode 10 and the inner electrode 20 protrude from the distal end side of the rubber-coated part 32. This allows the outer electrode 10 and the inner electrode 20 to better press against oral lesion tissues via the first tissue-contacting functional surface and the second tissue-contacting functional surface, thereby improving the efficiency of pulse ablation treatment.

In the embodiments of this application, the protrusion height of the outer electrode 10 from the distal end side of the rubber-coated part 32 is 0.1-0.3 mm, and the protrusion height of the inner electrode 20 from the distal end side of the rubber-coated part 32 is also 0.1-0.3 mm. This further enhances the pressing and fitting effect of the outer electrode 10 and the inner electrode 20 against oral lesion tissues, further improving the efficiency of pulse ablation treatment.

In some specific embodiments, the protrusion height of the outer electrode 10 from the distal end side of the rubber-coated part 32 is 0.2 mm, and the protrusion height of the inner electrode 20 from the distal end side of the rubber-coated part 32 is also 0.2 mm.

Furthermore, the protrusion height of the outer electrode 10 from the distal end side of the rubber-coated part 32 is consistent with that of the inner electrode 20 from the distal end side of the rubber-coated part 32.

In the embodiments of this application, the operating rod 40 comprises a first sub-housing 41 and a second sub-housing 42 that are fixedly connected. The proximal end of the electrode fixing base 31 is fixedly arranged between the first sub-housing 41 and the second sub-housing 42. The distal end of at least one of the first sub-housing 41 and the second sub-housing 42 is fixedly connected to the electrode fixing base 31 and extends into the rubber-coated part 32.

Furthermore, after the first sub-housing 41 and the second sub-housing 42 are fixedly connected, they form an operating rod cavity. The proximal end of the electrode fixing base 31 extends into the operating rod cavity and is fixed between the first sub-housing 41 and the second sub-housing 42. At least one of the first sub-housing 41 and the second sub-housing 42 extends into the rubber-coated part 32 and is fixedly connected to the electrode fixing base 31.

Specifically, the first sub-housing 41 and the second sub-housing 42 are engaged through a pin-in-hole fit and can be fixedly connected by laser welding.

In some embodiments, the proximal end of the electrode fixing base 31 can extend into the operating rod cavity and be fixedly clamped between the first sub-housing 41 and the second sub-housing 42, which can improve the reliability of the fixed connection between the electrode fixing base 31 and the operating rod 40.

Specifically, the distal end of the first sub-housing 41 protrudes relative to the distal end of the second sub-housing 42, and the distal end of the first sub-housing 41 is fixedly connected to the electrode fixing base 31. Specifically, the rubber-coated part 32 can wrap and fix the distal end of the electrode fixing base 31 and the distal end of the first sub-housing 41, with the distal end of the first sub-housing 41 extending into the rubber-coated part 32, which can improve the reliability of the fixed connection between the first sub-housing 41 and the electrode fixing base 31.

In some embodiments, the distal end of the first sub-housing 41 and the electrode fixing base 31 are engaged through a pin-in-hole fit, and the distal end of the first sub-housing 41 and the electrode fixing base 31 can be fixedly connected by laser welding.

In the embodiments of this application, an outer electrode positioning structure 311 is arranged on the distal end side of the electrode fixing base 31, and the outer electrode 10 is fixedly connected to the outer electrode positioning structure 311. The outer electrode positioning structure 311 has an outer electrode abutment surface 3111, and at least one first recessed groove 3112 is provided in a depressed manner on the outer electrode abutment surface 3111.

In some embodiments, the outer electrode 10 is fixedly connected to the outer electrode abutment surface 3111 by adhesive bonding. This results in a simple structure and a high connection strength between the outer electrode 10 and the outer electrode abutment surface 3111.

Furthermore, the outer electrode positioning structure 311 also has an outer electrode positioning surface 3113, which intersects with the outer electrode abutment surface 3111.

Specifically, the outer electrode positioning surface 3113 is used to restrict the movement of the outer electrode 10 along its radial direction.

Preferably, the outer electrode positioning surface 3113 is arranged perpendicular to the outer electrode abutment surface 3111.

In the embodiments of this application, the outer electrode 10 is fixedly connected to the outer electrode positioning structure 311 via the outer electrode abutment surface 3111 and the outer electrode positioning surface 3113. This arrangement restricts the movement of the outer electrode 10 relative to the electrode fixing base 31, facilitating the injection molding of the rubber-coated part 32.

In some embodiments, multiple first recessed grooves 3112 are provided in a depressed manner on the outer electrode abutment surface 3111. The provision of the first recessed grooves 3112 can reduce the thermal expansion and contraction of the outer electrode abutment surface 3111 during the injection molding of the rubber-coated part 32. It can also increase the adhesive area between the adhesive and the outer electrode 10 when the outer electrode 10 is bonded to the outer electrode abutment surface 3111, thereby enhancing the adhesive force that secures the outer electrode 10 to the outer electrode positioning structure 311. This prevents the outer electrode 10 from detaching during pulse ablation treatment, improving the operational safety of the pulse ablation device.

In some specific embodiments, four first recessed grooves 3112 are provided in a depressed manner on the outer electrode abutment surface 3111.

It should be noted that the number of first recessed grooves 3112 is determined according to actual circumstances, and this application does not impose any limitations on the number of first recessed grooves 3112.

In the embodiments of this application, an inner electrode positioning structure 312 is provided on the distal end side of the electrode fixing base 31, and the inner electrode 20 is fixedly connected to the inner electrode positioning structure 312. The inner electrode positioning structure 312 has an inner electrode abutment surface 3121, with at least one second recessed groove 3122 provided in a depressed manner on the inner electrode abutment surface 3121.

In some embodiments, the inner electrode 20 is fixedly connected to the inner electrode abutment surface 3121 by adhesive bonding, resulting in a simple structure and a high connection strength between the inner electrode 20 and the inner electrode abutment surface 3121.

Furthermore, the inner electrode positioning structure 312 also has an inner electrode positioning surface 3123 that intersects with the inner electrode abutment surface 3121.

Specifically, the inner electrode positioning surface 3123 is configured to restrict the radial movement of the inner electrode 20.

Preferably, the inner electrode positioning surface 3123 is set perpendicular to the inner electrode abutment surface 3121.

In the embodiments of this application, the inner electrode 20 is fixedly connected to the inner electrode positioning structure 312 via the inner electrode abutment surface 3121 and the inner electrode positioning surface 3123. This arrangement restricts the movement of the inner electrode 20 relative to the electrode fixing base 31, facilitating the injection molding of the rubber-coated part 32.

In some embodiments, multiple second recessed grooves 3122 are provided in a depressed manner on the inner electrode abutment surface 3121. The provision of the second recessed grooves 3122 can reduce the thermal expansion and contraction of the inner electrode abutment surface 3121 during the injection molding of the rubber-coated part 32. It can also increase the adhesive area between the adhesive and the inner electrode 20 when the inner electrode 20 is bonded to the inner electrode abutment surface 3121, thereby enhancing the adhesive force that secures the inner electrode 20 to the inner electrode positioning structure 312. This prevents the inner electrode 20 from detaching during pulse ablation treatment, improving the operational safety of the pulse ablation device.

In some specific embodiments, two second recessed grooves 3122 are provided in a depressed manner on the inner electrode abutment surface 3121.

It should be noted that the number of second recessed grooves 3122 is determined according to actual circumstances, and this application does not impose any limitations on the number of second recessed grooves 3122.

In the embodiments of this application, the outer electrode positioning structure 311 is arranged on the outer peripheral side of the inner electrode positioning structure 312, with a gap between them. There is a first gap 313 between the outer electrode positioning structure 311 and the inner electrode positioning structure 312. The outer electrode positioning structure 311 is provided with at least one through-hole 314 that penetrates through the first gap 313.

Specifically, the rubber-coated part 32 can extend into the through-hole 314 and the first gap 313, thereby increasing the contact area between the rubber-coated part 32 and the electrode fixing base 31 and enhancing the adhesion of the rubber-coated part 32.

In some specific embodiments, the outer electrode positioning structure 311 is provided with five through-holes 314.

It should be noted that the number of through-holes 314 is determined according to actual circumstances, and this application does not impose any limitations on the number of through-holes 314.

In the embodiments of this application, the pulse ablation device also includes an outer electrode connecting wire and an inner electrode connecting wire. The outer electrode connecting wire is fixedly connected to the outer electrode 10, and the inner electrode connecting wire is fixedly connected to the inner electrode 20. The electrode fixing base 31 is provided with at least one wire retaining slot 315, and the outer electrode connecting wire and the inner electrode connecting wire are respectively engaged with the wire retaining slot 315. This arrangement better secures the outer electrode connecting wire and the inner electrode connecting wire, preventing their movement within the electrode fixing base 31, detachment of the outer electrode connecting wire from the outer electrode 10, and detachment of the inner electrode connecting wire from the inner electrode 20.

In the embodiments of this application, the electrode fixing base 31 is provided with a first wire routing hole 316, and the operating rod 40 is provided with a second wire routing hole 43 that is aligned with the first wire routing hole 316. The wire retaining slot 315 is arranged on the first wire routing hole 316. Both the first wire routing hole 316 and the second wire routing hole 43 are used to accommodate the outer electrode connecting wire and the inner electrode connecting wire.

Specifically, the provision of the first wire routing hole 316 facilitates the routing of the outer electrode connecting wire and the inner electrode connecting wire, and also allows the rubber-coated part 32 to extend into it, thereby increasing the contact area between the rubber-coated part 32 and the electrode fixing base 31 and enhancing the adhesion of the rubber-coated part 32.

Furthermore, the second wire routing hole 43 is smoothly arranged to prevent bending of the outer electrode connecting wire and the inner electrode connecting wire within it, avoiding stress concentration caused by assembly tension or loosening of the wires, thereby preventing their fracture.

In the embodiments of this application, the electrode fixing device 30 has a curved section, and the angle between the curved section of the electrode fixing device 30 and the axial direction of the operating rod 40 is 35-50°. This arrangement improves the insertion flexibility of the pulse ablation device and enhances the adherence of the distal end sides of the rubber-coated part 32, the outer electrode 10, and the inner electrode 20 to oral lesion tissues.

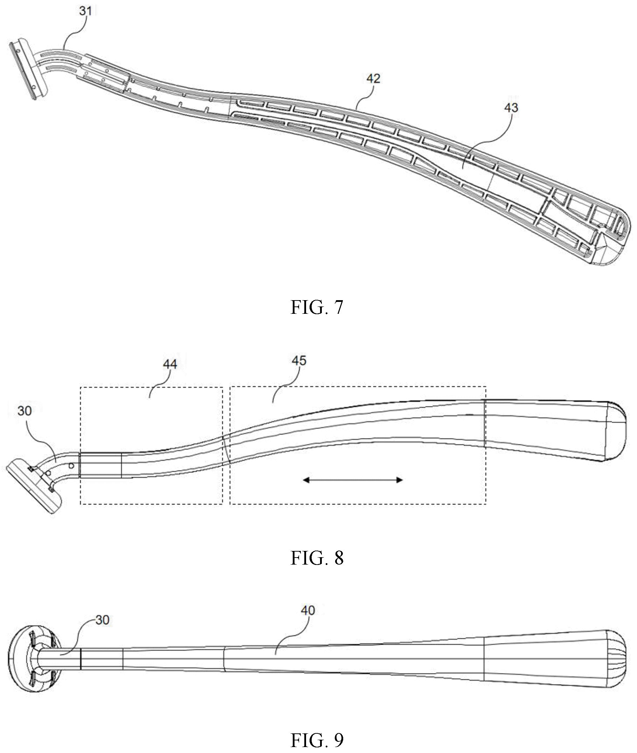

It should be noted that, as indicated by the arrow direction in FIG. 8, the axial direction of the operating rod 40 can be the directional axis indicating the height of the pulse ablation device in its longitudinal section.

Preferably, the angle between the curved section of the electrode fixing device 30 and the axial direction of the operating rod 40 is 45°.

In the embodiments of this application, the operating rod 40 includes multiple operating zones, each with a curved section. The curved sections of adjacent operating zones bend in different directions. The curved section of the operating zone closest to the electrode fixing device 30 bends in a different direction from that of the electrode fixing device 30. This arrangement further improves the insertion flexibility of the pulse ablation device and enhances the adherence of the distal end sides of the rubber-coated part 32, the outer electrode 10, and the inner electrode 20 to oral lesion tissues.

In the embodiments of this application, the multiple operating zones include a first operation zone 44 and a second operation zone 45, with the first operation zone 44 located close to the electrode fixing device 30. The second operation zone 45, the first operation zone 44, and the electrode fixing device 30 are sequentially connected in an S-shape.

Specifically, the curved section of the first operation zone 44 bends in a different direction from that of the electrode fixing device 30, and the curved section of the first operation zone 44 bends in a different direction from that of the second operation zone 45.

In the embodiments of this application, the sequentially connected S-shaped second operation zone 45, first operation zone 44, and electrode fixing device 30 further improve the insertion flexibility of the pulse ablation device and enhance the adherence of the distal end sides of the rubber-coated part 32, the outer electrode 10, and the inner electrode 20 to oral lesion tissues.

In the embodiments of this application, the angle between the first operation zone 44 and the axial direction of the operating rod 40 is 15-25°, and the angle between the second operation zone 45 and the axial direction of the operating rod 40 is also 15-25°, which optimizes the insertion flexibility of the pulse ablation device.

Preferably, the angle between the first operation zone 44 and the axial direction of the operating rod 40 is 20°, and the angle between the second operation zone 45 and the axial direction of the operating rod 40 is also 20°.

In the embodiments of this application, the arc-shaped curvature of the second operation zone 45, the first operation zone 44, and the electrode fixing device 30 facilitates the more flexible insertion of the distal end of the pulse ablation device into corner positions within the oral cavity, enabling more precise and better-adhered pressing onto oral lesion tissues. This arc-shaped curvature also facilitates the operator's grip, allowing the operator to change the grip position of the pulse ablation device according to the location of the oral lesion tissues, thereby improving the operator's experience.

In the embodiments of this application, the outer electrode 10 is ring-shaped, and the inner electrode 20 is circular; the outer electrode 10 and the inner electrode 20 are concentrically arranged. This arrangement improves the uniformity of pulse emission by the electrode pair composed of the outer electrode 10 and the inner electrode 20, facilitates their assembly, and simplifies the structure of the pulse ablation device.

Furthermore, both the outer electrode 10 and the inner electrode 20 are sheet-like electrodes, facilitating their adherence and fixation to the electrode fixing base 31, and enhancing the fixing effects of the electrode fixing base 31 and the rubber-coated part 32 on the outer electrode 10 and the inner electrode 20, thereby further preventing their displacement and detachment.

Hereinafter, specific embodiments of this application are introduced based on the above technical solutions.

### Embodiment 2

Referring to Figures 1-9, Embodiment 2 provides a pulse ablation device for application to oral lesion tissues, comprising an outer electrode 10, an inner electrode 20, an electrode fixing device 30, and an operating rod 40. The outer electrode 10 is fixedly connected to the electrode fixing device 30, and the inner electrode 20 is also fixedly connected to the electrode fixing device 30. The electrode fixing device 30 is fixedly connected to the operating rod 40. Both the outer electrode 10 and the inner electrode 20 are arranged at the distal end of the electrode fixing device 30. The outer electrode 10 is arranged to encircle the outer peripheral side of the inner electrode 20, and a discharge gap is formed between the outer electrode 10 and the inner electrode 20, which can be broken down by current to form pulses.

The outer electrode 10 and the inner electrode 20 are respectively fixedly connected to the distal end of the electrode fixing device 30, and the electrode fixing device 30 is used to fix the relative positions of the outer electrode 10 and the inner electrode 20.

The outer electrode 10 has a ring shape, and the inner electrode 20 has a circular shape. The outer electrode 10 and the inner electrode 20 are concentrically arranged.

Both the outer electrode 10 and the inner electrode 20 are sheet electrodes.

The protrusion height of the outer electrode 10 from the distal end side of the rubber-coated part 32 is 0.2 mm, and the protrusion height of the inner electrode 20 from the distal end side of the rubber-coated part 32 is also 0.2 mm.

The outer electrode 10 and the inner electrode 20 form an electrode pair that emits pulses to oral lesion tissues for pulse ablation therapy of the oral lesion tissues.

The distal end of the outer electrode 10 has a first tissue-abutting functional surface, which is used for abutting against oral lesion tissues. Correspondingly, the distal end of the inner electrode 20 has a second tissue-abutting functional surface, which is used for abutting against oral lesion tissues.

The outer electrode 10 and the inner electrode 20 can form an electrode pair that emits pulses with a microsecond-level width to oral lesion tissues, thereby damaging the stability of the cell membrane surfaces of the oral lesion tissues, causing multiple hydrophilic micropores to appear on the cell surfaces of the oral lesion tissues, disrupting the cellular homeostasis of the oral lesion tissues, and leading to the death of the oral lesion tissue cells, thus achieving the therapeutic effect of pulse ablation of oral lesion tissues.

The electrode fixing device 30 comprises an electrode fixing base 31 and a rubber-coated part 32. The electrode fixing base 31 is fixedly connected to the operating rod 40. The proximal ends of the outer electrode 10 and the inner electrode 20 are both fixedly connected to the distal end of the electrode fixing base 31. The rubber-coated part 32 is wrapped around and arranged at the distal end of the electrode fixing base 31, and is used to fix the outer electrode 10 and the inner electrode 20 onto the electrode fixing base 31.

The electrode fixing base 31 is fixedly connected to the distal end of the operating rod 40.

The electrode fixing base 31 is in clearance-pin fit with the operating rod 40, and the electrode fixing base 31 and the operating rod 40 are fixedly connected by laser welding.

The proximal end of the outer electrode 10 is fixedly connected to the distal end of the electrode fixing base 31. Correspondingly, the proximal end of the inner electrode 20 is fixedly connected to the distal end of the electrode fixing base 31.

The rubber-coated part 32 is sleeved over the electrode fixing base 31 and covers the connecting portions between the outer electrode 10 and the electrode fixing base 31 and between the inner electrode 20 and the electrode fixing base 31. The rubber-coated part 32 is used to fix the outer electrode 10 and the inner electrode 20 respectively at the end side of the electrode fixing base 31. Both the outer electrode 10 and the inner electrode 20 protrude outward from the end side of the rubber-coated part 32.

The electrode fixing base 31 and the rubber-coated part 32 cooperate to fix the outer electrode 10 and the inner electrode 20.

The rubber-coated part 32 can be a soft rubber encapsulation. Through injection molding, the rubber-coated part 32 is wrapped and fixed onto the electrode fixing base 31 to fix the outer electrode 10 and the inner electrode 20.

Both the outer electrode 10 and the inner electrode 20 protrude from the distal end side of the rubber-coated part 32.

The protrusion height of the outer electrode 10 from the distal end side of the rubber-coated part 32 is the same as that of the inner electrode 20 from the distal end side of the rubber-coated part 32.

An outer electrode positioning structure 311 is provided on the distal end side of the electrode fixing base 31, and the outer electrode 10 is fixedly connected to the outer electrode positioning structure 311. The outer electrode positioning structure 311 has an outer electrode abutting surface 3111.

The outer electrode 10 is fixedly connected to the outer electrode abutting surface 3111 by adhesive bonding.

The outer electrode positioning structure 311 also has an outer electrode positioning surface 3113, which is arranged perpendicular to the outer electrode abutting surface 3111.

The outer electrode positioning surface 3113 is used to restrict the movement of the outer electrode 10 along its radial direction.

The outer electrode 10 is fixedly connected to the outer electrode positioning structure 311 through the outer electrode abutting surface 3111 and the outer electrode positioning surface 3113.

Four first recessed grooves 3112 are recessed on the outer electrode abutting surface 3111.

An inner electrode positioning structure 312 is provided on the distal end side of the electrode fixing base 31, and the inner electrode 20 is fixedly connected to the inner electrode positioning structure 312. The inner electrode positioning structure 312 has an inner electrode abutting surface 3121.

The inner electrode 20 is fixedly connected to the inner electrode abutting surface 3121 by adhesive bonding.

The inner electrode positioning structure 312 also has an inner electrode positioning surface 3123, which is arranged perpendicular to the inner electrode abutting surface 3121.

The inner electrode positioning surface 3123 is used to restrict the movement of the inner electrode 20 along its radial direction.

The inner electrode 20 is fixedly connected to the inner electrode positioning structure 312 through the inner electrode abutting surface 3121 and the inner electrode positioning surface 3123.

Two second recessed grooves 3122 are recessed on the inner electrode abutting surface 3121.

The outer electrode positioning structure 311 is arranged on the outer peripheral side of the inner electrode positioning structure 312, and the outer electrode positioning structure 311 is spaced apart from the inner electrode positioning structure 312, with a first gap 313 between them. Five through-holes 314 are provided on the outer electrode positioning structure 311, and the through-holes 314 are in communication with the first gap 313.

The rubber-coated part 32 can extend into the through-holes 314 and the first gap 313.

The operating rod 40 comprises a first sub-housing 41 and a second sub-housing 42 that are fixedly connected. The proximal end of the electrode fixing base 31 is fixedly arranged between the first sub-housing 41 and the second sub-housing 42. The distal end of at least one of the first sub-housing 41 and the second sub-housing 42 is fixedly connected to the electrode fixing base 31 and extends into the rubber-coated part 32.

After the first sub-housing 41 and the second sub-housing 42 are fixedly connected, an operating rod cavity is formed. The proximal end of the electrode fixing base 31 extends into the operating rod cavity and is fixed between the first sub-housing 41 and the second sub-housing 42. At least one of the first sub-housing 41 and the second sub-housing 42 extends into the rubber-coated part 32 and is fixedly connected to the electrode fixing base 31.

The first sub-housing 41 and the second sub-housing 42 are in clearance-pin fit, and can be fixedly connected by laser welding.

The proximal end of the electrode fixing base 31 can extend into the operating rod cavity and is fixedly clamped between the first sub-housing 41 and the second sub-housing 42.

The distal end of the first sub-housing 41 protrudes relative to the distal end of the second sub-housing 42, and the distal end of the first sub-housing 41 is fixedly connected to the electrode fixing base 31. Specifically, the rubber-coated part 32 can wrap and fix the distal end of the electrode fixing base 31 and the distal end of the first sub-housing 41, with the distal end of the first sub-housing 41 extending into the rubber-coated part 32.

The distal end of the first sub-housing 41 and the electrode fixing base 31 are in clearance-pin fit, and can be fixedly connected by laser welding.

The pulse ablation device also includes an outer electrode connecting wire and an inner electrode connecting wire. The outer electrode connecting wire is fixedly connected to the outer electrode 10, and the inner electrode connecting wire is fixedly connected to the inner electrode 20. A wire connecting slot 315 is provided on the electrode fixing base 31, and the outer electrode connecting wire and the inner electrode connecting wire are respectively engaged with the wire connecting slot 315.

A first wire routing hole 316 is provided on the electrode fixing base 31, and a second wire routing hole 43 is provided inside the operating rod 40. The first wire routing hole 316 is in communication with the second wire routing hole 43, and the wire connecting slot 315 is arranged on the first wire routing hole 316. Both the first wire routing hole 316 and the second wire routing hole 43 are used to accommodate the outer electrode connecting wire and the inner electrode connecting wire.

The provision of the first wire routing hole 316 facilitates the routing of the outer electrode connecting wire and the inner electrode connecting wire, and also allows the rubber-coated part 32 to extend in.

The second wire routing hole 43 is smoothly arranged to prevent the bending of the outer electrode connecting wire and the inner electrode connecting wire inside the second wire routing hole 43, and to avoid stress concentration caused by assembly pulling or loosening of the outer electrode connecting wire and the inner electrode connecting wire.

The electrode fixing device 30 has a curved section, and the angle between the curved section of the electrode fixing device 30 and the axial direction of the operating rod 40 is 45°.

The operating rod 40 includes multiple operating sections, which include curved sections. The curved sections of adjacent operating sections bend in different directions. The curved section of the operating section close to the electrode fixing device 30 among the multiple operating sections bends in a different direction from the curved section of the electrode fixing device 30.

The multiple operating sections include a first operation zone 44 and a second operation zone 45, with the first operation zone 44 being close to the electrode fixing device 30. The second operation zone 45, the first operation zone 44, and the electrode fixing device 30 are sequentially connected in an S-shape.

The curved section of the first operation zone 44 bends in a different direction from the curved section of the electrode fixing device 30. The curved section of the first operation zone 44 bends in a different direction from the curved section of the second operation zone 45.

The angle between the first operation zone 44 and the axial direction of the operating rod 40 is 20°. The angle between the second operation zone 45 and the axial direction of the operating rod 40 is 20°.

### Embodiment 3

The difference between Embodiment 3 and Embodiment 2 lies in the protruding arrangements of the outer electrode 10 and the inner electrode 20. The similarities between Embodiment 3 and Embodiment 2 will not be elaborated on here. The differences between Embodiment 3 and Embodiment 2 are described as follows:
The outer electrode 10 has an annular shape, and the inner electrode 20 has a circular shape. The outer electrode 10 and the inner electrode 20 are concentrically arranged.

Both the outer electrode 10 and the inner electrode 20 are sheet-like electrodes.

The outer electrode 10 protrudes from the distal end side of the rubber-coated part 32 by a height of 0.15 mm, and the inner electrode 20 protrudes from the distal end side of the rubber-coated part 32 by a height of 0.15 mm.

The outer electrode 10 and the inner electrode 20 form an electrode pair, which emits pulses to oral lesion tissues for pulse ablation treatment of the oral lesion tissues.

### Embodiment 4

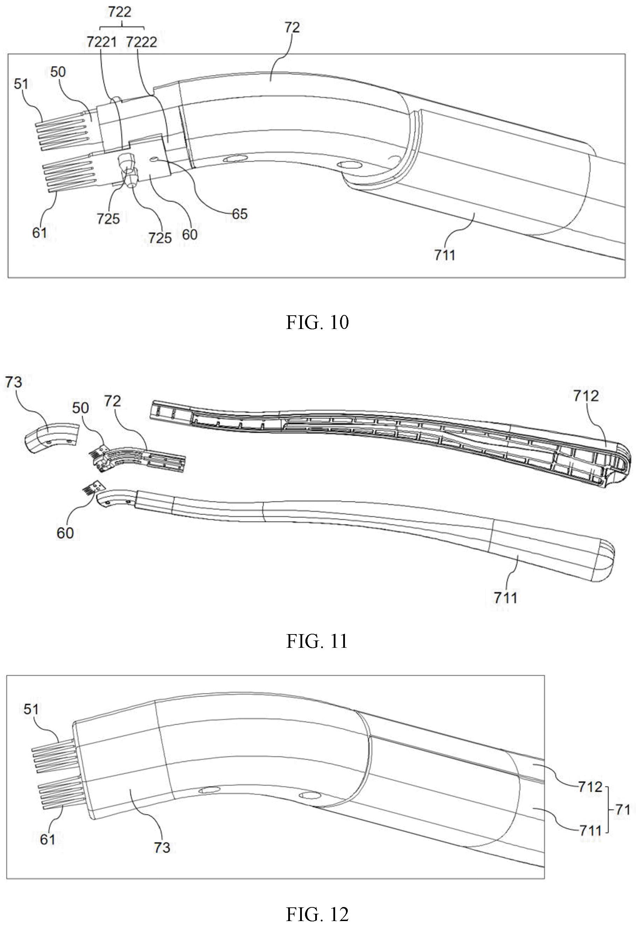

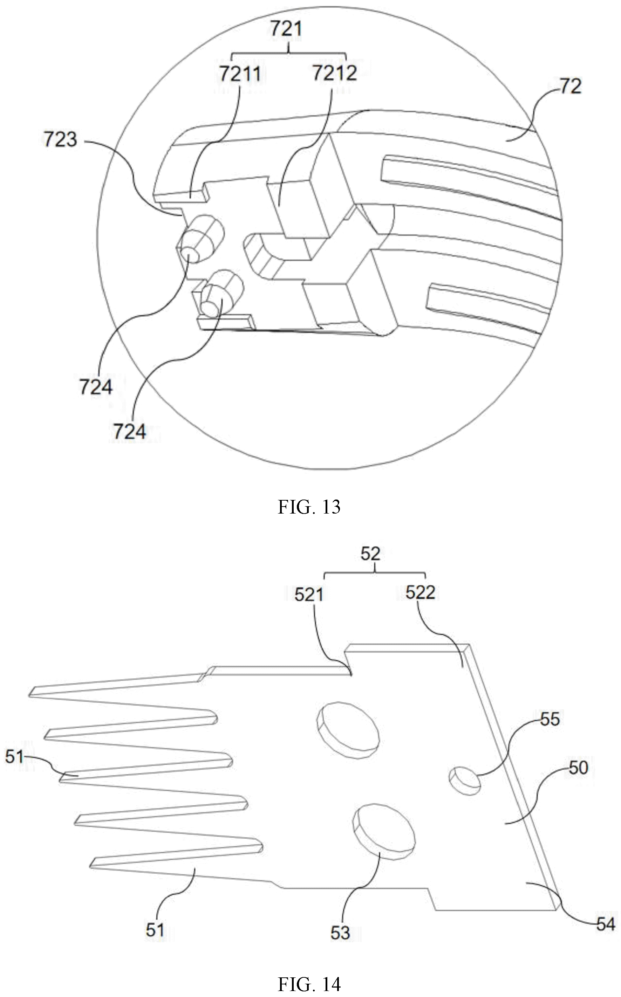

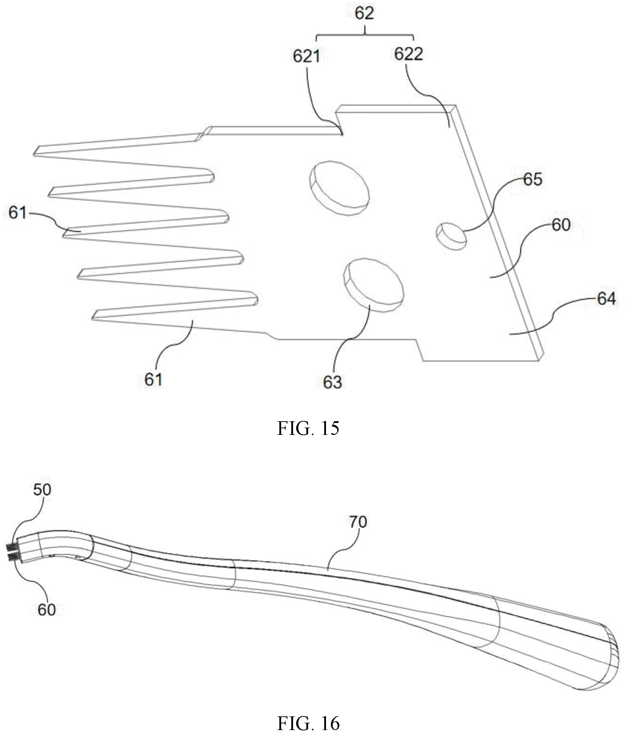

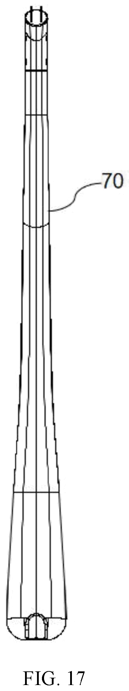

Referring to FIGS. 10-18, an embodiment of the present application provides an oral ablation device, which includes a first electrode 50, a second electrode 60, and an operating handle 70. The first electrode 50 is fixedly connected to the operating handle 70, and the second electrode 60 is also fixedly connected to the operating handle 70. The first electrode 50 and the second electrode 60 are arranged opposite to each other. The first electrode 50 has at least one first microneedle 51, and the second electrode 60 has at least one second microneedle 61. Both the first microneedles 51 and the second microneedles 61 extend from the end side of the operating handle 70. The first electrode 50 and the second electrode 60 are used to emit pulses to target tissues through the first microneedles 51 and the second microneedles 61.

In some embodiments, the first electrode 50 and the second electrode 60 are respectively fixedly connected to the distal end of the operating handle 70, and the operating handle 70 is used to fix the relative positions of the first electrode 50 and the second electrode 60.

Preferably, the structure and connection mode of the first electrode 50 are consistent with those of the second electrode 60. In this way, the structure and assembly of the oral ablation device can be simplified.

In some embodiments, the first electrode 50 has multiple first microneedles 51, and the second electrode 60 has multiple second microneedles 61. The provision of multiple first microneedles 51 and multiple second microneedles 61 can enhance the energy of pulses emitted by the first electrode 50 and the second electrode 60, thereby improving the ablation treatment efficiency of the oral ablation device.

Preferably, the number of the first microneedles 51 is the same as that of the second microneedles 61.

In some embodiments, the first microneedles 51 are used to puncture and extend into target tissues, and correspondingly, the second microneedles 61 are also used to puncture and extend into target tissues. The first electrode 50 and the second electrode 60 form an electrode pair, and emit pulses within the target tissues through the first microneedles 51 and the second microneedles 61 to perform ablation treatment on the target tissues.

Specifically, the first electrode 50 and the second electrode 60 can emit pulses with a width at the microsecond or nanosecond level to the target tissues through the first microneedles 51 and the second microneedles 61, so as to disrupt the stability of the cell membrane surface of the target tissues, creating multiple hydrophilic micropores on the surface, thereby disrupting cellular homeostasis and ultimately leading to cell death, achieving the ablation treatment effect on the target tissues.

Specifically, since different tissues have different energy thresholds, by controlling the energy of pulses emitted by the first microneedles 51 and the second microneedles 61, the pulses emitted by the first microneedles 51 and the second microneedles 61 can be precisely targeted to the desired location, exhibiting tissue selectivity. This enables pulse ablation treatment to be performed on the target tissues without damaging other tissues.

The oral ablation device of the present application comprises a first electrode, a second electrode, and an operating handle. The first electrode is fixedly connected to the operating handle, the second electrode is also fixedly connected to the operating handle, and the first electrode and the second electrode are arranged opposite to each other. The first electrode has at least one first microneedle, the second electrode has at least one second microneedle, and both the first microneedles and the second microneedles extend from the end side of the operating handle. The first electrode and the second electrode are used to emit pulses to target tissues through the first microneedles and the second microneedles. The oral ablation device of the present application can selectively treat oral lesion tissues and improve the treatment effect of oral ablation by emitting pulses to target tissues through at least one first microneedle and at least one second microneedle extending from the end side of the operating handle.

In the embodiments of the present application, the operating handle 70 includes a support housing 71 and an electrode adapter 72. The electrode adapter 72 is fixedly connected to the end side of the support housing 71. One end of the first electrode 50, which is opposite to the tip of the first microneedle 51, and one end of the second electrode 60, which is opposite to the tip of the second microneedle 61, are respectively fixedly connected to the end of the electrode adapter 72 that is away from the support housing 71. The first electrode 50 and the second electrode 60 are fixed on opposite end sides of the end of the electrode adapter 72 that is away from the support housing 71.

In some embodiments, the electrode adapter 72 is in a hole-pin fit with the support housing 71, and the electrode adapter 72 and the support housing 71 are fixedly connected by laser welding.

Specifically, the proximal end of the first electrode 50 is fixedly connected to the distal end of the electrode adapter 72. Correspondingly, the proximal end of the second electrode 60 is fixedly connected to the distal end of the electrode adapter 72.

Specifically, the first electrode 50 and the second electrode 60 are arranged on opposite sides of the electrode adapter 72, and there is a preset spacing distance between the first electrode 50 and the second electrode 60.

In the embodiments of the present application, the first electrode 50 and the second electrode 60 are fixedly connected to opposite sides of the electrode adapter 72 at a preset spacing distance, which can improve the reliability of the fixed connections between the operating handle 70 and the first electrode 50, and between the operating handle 70 and the second electrode 60.

In the embodiments of the present application, the operating handle 70 further includes an electrode fixing part 73. The electrode fixing part 73 is sleeved over the electrode adapter 72 and covers the connecting portion between the first electrode 50 and the electrode adapter 72 and the connecting portion between the second electrode 60 and the electrode adapter 72. The electrode fixing part 73 is used to fix the first electrode 50 and the second electrode 60 respectively to the end side of the electrode adapter 72. Both the first microneedle 51 and the second microneedle 61 extend outward from the end side of the electrode fixing part 73.

Specifically, the electrode adapter 72 and the electrode fixing part 73 cooperate to fix the first electrode 50 and the second electrode 60.

In some embodiments, the electrode fixing part 73 can be a soft rubber coating. Through injection molding, the electrode fixing part 73 is wrapped and fixed on the electrode adapter 72 to fix the first electrode 50 and the second electrode 60.

In some embodiments, both the first microneedle 51 and the second microneedle 61 fully extend from the end side of the electrode fixing part 73.

In the embodiments of the present application, setting the electrode fixing part 73 as a soft rubber coating wrapped around the end side of the electrode adapter 72 can improve the fixing effect on the first electrode 50 and the second electrode 60, prevent the first electrode 50 and/or the second electrode 60 from falling off during the oral ablation process, and improve the operational safety of the oral ablation device.

In the embodiments of the present application, the support housing 71 includes a first housing 711 and a second housing 712. After the first housing 711 and the second housing 712 are fixedly connected, a housing inner cavity is formed. One end of the electrode adapter 72 extends into the housing inner cavity and is fixed between the first housing 711 and the second housing 712. At least one of the first housing 711 and the second housing 712 extends into the electrode fixing part 73 and is fixedly connected to the electrode adapter 72.

In some embodiments, the first housing 711 and the second housing 712 are in a hole-pin fit, and the first housing 711 and the second housing 712 can be fixedly connected by laser welding.

Specifically, the proximal end of the electrode adapter 72 can extend into the housing inner cavity and be fixedly clamped between the first housing 711 and the second housing 712. In this way, the reliability of the fixed connection between the electrode adapter 72 and the support housing 71 can be improved.

In some embodiments, the distal end of the first housing 711 protrudes relative to the distal end of the second housing 712, and the distal end of the first housing 711 is fixedly connected to the electrode adapter 72. Specifically, the electrode fixing part 73 can wrap and fix the distal end of the electrode adapter 72 and the distal end of the first housing 711. The distal end of the first housing 711 extends into the electrode fixing part 73. In this way, the reliability of the fixed connection between the first housing 711 and the electrode adapter 72 can be improved.

Specifically, the distal end of the first housing 711 and the electrode adapter 72 are in a hole-pin fit, and the distal end of the first housing 711 and the electrode adapter 72 can be fixedly connected by laser welding.

In the embodiments of the present application, the end part of the electrode adapter 72 has a first electrode positioning portion 721 and a second electrode positioning portion 722. The first electrode 50 has a third electrode positioning portion 52 that is compatible with the first electrode positioning portion 721. The first electrode positioning portion 721 and the third electrode positioning portion 52 can abut against each other to restrict the movement of the first electrode 50. The second electrode 60 has a fourth electrode positioning portion 62 that is compatible with the second electrode positioning portion 722. The second electrode positioning portion 722 and the fourth electrode positioning portion 62 can abut against each other to restrict the movement of the second electrode 60.

In some embodiments, the first electrode positioning portion 721 includes a first stop part 3211 and a second stop part 3212. Correspondingly, the third electrode positioning portion 52 includes a third stop part 121 that is compatible with the first stop part 3211 and a fourth stop part 122 that is compatible with the second stop part 3212. The first stop part 3211 and the third stop part 121 cooperate to abut against each other, and the second stop part 3212 and the fourth stop part 122 cooperate to abut against each other, which can restrict the movement of the first electrode 50 relative to the electrode adapter 72 and facilitate the injection molding and rubber coating of the electrode fixing part 73.

In some embodiments, the second electrode positioning portion 722 includes a fifth stop part 3221 and a sixth stop part 3222. Correspondingly, the fourth electrode positioning portion 62 includes a seventh stop part 221 that is compatible with the fifth stop part 3221 and an eighth stop part 222 that is compatible with the sixth stop part 3222. The fifth stop part 3221 and the seventh stop part 221 cooperate to abut against each other, and the sixth stop part 3222 and the eighth stop part 222 cooperate to abut against each other, which can restrict the movement of the second electrode 60 relative to the electrode adapter 72 and facilitate the injection molding and rubber coating of the electrode fixing part 73.

In the embodiments of the present application, the end part of the electrode adapter 72 is provided with grooves 723, and the electrode fixing part 73 can extend into the grooves 723.

Specifically, multiple grooves 723 are recessed at the end part of the electrode adapter 72. In this way, the contact area between the electrode fixing part 73 and the electrode adapter 72 can be increased, and the firmness of the rubber coating of the electrode fixing part 73 can be improved.

In the embodiments of the present application, the end part of the electrode adapter 72 also has a first electrode assembly pin 724 and a second electrode assembly pin 725. The first electrode 50 has a first electrode assembly hole 53 that is compatible with the first electrode assembly pin 724, and the first electrode assembly pin 724 can extend through the first electrode assembly hole 53. The second electrode 60 has a second electrode assembly hole 63 that is compatible with the second electrode assembly pin 725, and the second electrode assembly pin 725 can extend through the second electrode assembly hole 63.

Specifically, the first electrode 50 is sleeved over the first electrode assembly pin 724 through the first electrode assembly hole 53. In this way, the assembly convenience of the first electrode 50 can be improved. The protruding setting of the first electrode assembly pin 724 can also improve the firmness of the rubber coating of the electrode fixing part 73.

Specifically, the second electrode 60 is sleeved over the second electrode assembly pin 725 through the second electrode assembly hole 63. In this way, the assembly convenience of the second electrode 60 can be improved. The protruding setting of the second electrode assembly pin 725 can also improve the firmness of the rubber coating of the electrode fixing part 73.

In the embodiments of the present application, the first electrode 50 includes multiple first microneedles 51 and a first electrode connecting portion 54 that is fixedly connected to the multiple first microneedles 51. The multiple first microneedles 51 are adjacent to each other and arranged on the end side of the first electrode connecting portion 54, and a first arc-shaped connecting structure is formed between adjacent first microneedles 51. The second electrode 60 includes multiple second microneedles 61 and a second electrode connecting portion 64 that is fixedly connected to the multiple second microneedles 61. The multiple second microneedles 61 are adjacent to each other and arranged on the end side of the second electrode connecting portion 64, and a second arc-shaped connecting structure is formed between adjacent second microneedles 61.

Specifically, the multiple first microneedles 51 are adjacent to each other and arranged on the distal end side of the first electrode connecting portion 54. Adjacent first microneedles 51 are smoothly connected by the first arc-shaped connecting structure. In this way, the stress concentration at the connecting portion between adjacent first microneedles 51 can be reduced, thereby reducing the possibility of the first microneedles 51 being bent by external forces.

Specifically, the multiple second microneedles 61 are adjacent to each other and arranged on the distal end side of the second electrode connecting portion 64. Adjacent second microneedles 61 are smoothly connected by the second arc-shaped connecting structure. In this way, the stress concentration at the connecting portion between adjacent second microneedles 61 can be reduced, thereby reducing the possibility of the second microneedles 61 being bent by external forces.

In some specific embodiments, the first electrode 50 includes five first microneedles 51. Correspondingly, the second electrode 60 includes five second microneedles 61.

It should be noted that the number of the first microneedles 51 and the number of the second microneedles 61 are determined according to the actual situation, and this application does not limit the number of the first microneedles 51 and the number of the second microneedles 61.

In the embodiments of the present application, the cross-sectional dimension of the distal end of the first microneedle 51 is smaller than the cross-sectional dimension of the proximal end of the first microneedle 51. The cross-sectional dimension of the distal end of the second microneedle 61 is smaller than the cross-sectional dimension of the proximal end of the second microneedle 61.

Specifically, from the distal end to the proximal end of the first microneedle 51, the cross-sectional dimension of the first microneedle 51 increases. In this way, the structural strength of the first microneedle 51 can be improved, the connection strength between the first microneedle 51 and the first electrode connecting portion 54 can be improved, and thus the bending resistance of the first microneedle 51 can be enhanced.

Specifically, from the distal end to the proximal end of the second microneedle 61, the cross-sectional dimension of the second microneedle 61 increases. In this way, the structural strength of the second microneedle 61 can be improved, the connection strength between the second microneedle 61 and the second electrode connecting portion 64 can be improved, and thus the bending resistance of the second microneedle 61 can be enhanced.

In the embodiments of the present application, the first microneedle 51 includes a first conical end, and the first conical end is arranged at the distal end of the first microneedle 51. The second microneedle 61 includes a second conical end, and the second conical end is arranged at the distal end of the second microneedle 61.

In some embodiments, the shape of the first microneedle 51 is conical. In this way, the puncturing effect of the first microneedle 51 can be ensured, and the bending resistance of the first microneedle 51 can also be improved.

Correspondingly, the shape of the second microneedle 61 is conical. In this way, the puncturing effect of the second microneedle 61 can be ensured, and the bending resistance of the second microneedle 61 can also be improved.

In the embodiments of the present application, the operating handle 70 includes multiple operating areas connected in sequence. The operating area includes an arc-shaped section, and the arc-shaped sections of adjacent operating areas bend in different directions. In this way, the insertion flexibility of the oral ablation device can be improved, facilitating the first microneedle 51 and the second microneedle 61 to puncture and extend into the target tissue and improving the abutment degree between the distal end side of the electrode fixing part 73 and the target tissue.

In the embodiments of the present application, the multiple operating areas include a first gripping area 74, a second gripping area 75, and an insertion area 76. The first gripping area 74, the second gripping area 75, and the insertion area 76 are connected in sequence in an S-shape, and the insertion area 76 is respectively connected to the first electrode 50 and the second electrode 60.

Specifically, the arc-shaped section of the first gripping area 74 bends in a different direction from the arc-shaped section of the second gripping area 75, and the arc-shaped section of the second gripping area 75 bends in a different direction from the arc-shaped section of the insertion area 76.

Specifically, the first gripping area 74 is arranged at the proximal end of the support housing 71, the second gripping area 75 is arranged at the distal end of the support housing 71, and the insertion area 76 is arranged on the electrode fixing part 73.

In the embodiments of the present application, the first gripping area 74, the second gripping area 75, and the insertion area 76 connected in sequence in an S-shape can further improve the insertion flexibility of the oral ablation device and further improve the abutment degree between the distal end side of the electrode fixing part 73 and the target tissue.

In the embodiments of the present application, the oral ablation device has an operating handle axial direction. The first gripping area 74 and the operating handle axial direction have a first bending angle, and the first bending angle is 10-30°. The second gripping area 75 and the operating handle axial direction have a second bending angle, and the second bending angle is 10-30°. The insertion area 76 and the operating handle axial direction have a third bending angle, and the third bending angle is 30-55°. In this way, the insertion flexibility of the oral ablation device can be further improved, and the abutment degree between the distal end side of the electrode fixing part 73 and the target tissue can be further improved.

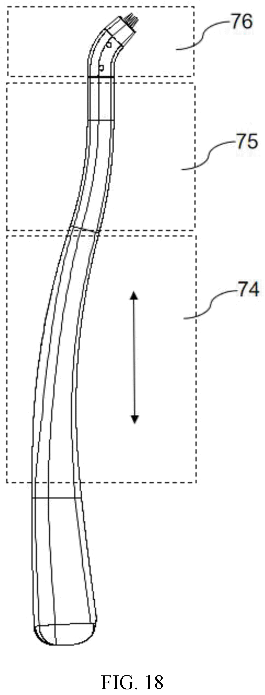

It can be understood that the operating handle axial direction can be a directional axis used to indicate the height of the oral ablation device on the longitudinal section of the oral ablation device. For example, please refer to the arrow direction in FIG. 18.

Preferably, the first bending angle between the first gripping area 74 and the operating handle axial direction is 20°, the second bending angle between the second gripping area 75 and the operating handle axial direction is 20°, and the third bending angle between the insertion area 76 and the operating handle axial direction is 45°.

In the embodiments of the present application, the arc-shaped bending settings of the first gripping area 74, the second gripping area 75, and the insertion area 76 facilitate the more flexible insertion of the distal end of the oral ablation device into corner positions in the oral cavity, facilitating the more precise and better-abutting pressing of the distal end of the oral ablation device onto the target positions in the oral cavity. The arc-shaped bending settings of the first gripping area 74, the second gripping area 75, and the insertion area 76 also facilitate the gripping by the operator and enable the operator to change the gripping position of the oral ablation device according to the position of the target tissue, which can improve the operator's operational experience.

In the embodiments of the present application, the oral ablation device further includes a first wire and a second wire. The first electrode 50 is provided with a first wire connection hole 55, which is used for the first wire to pass through and be fixedly connected to the first wire. The second electrode 60 is provided with a second wire connection hole 65, which is used for the second wire to pass through and be fixedly connected to the second wire.

In some embodiments, the first wire connection hole 55 penetrates through the first electrode 50. The first wire is fixedly connected to the first electrode 50 through the first wire connection hole 55. Specifically, the first wire and the first electrode 50 are welded at the first wire connection hole 55. In this way, the reliability of the fixed connection between the first wire and the first electrode 50 can be improved, and the falling off of the first wire can be prevented.

In some embodiments, the second wire connection hole 65 penetrates through the second electrode 60. The second wire is fixedly connected to the second electrode 60 through the second wire connection hole 65. Specifically, the second wire and the second electrode 60 are welded at the second wire connection hole 65. In this way, the reliability of the fixed connection between the second wire and the second electrode 60 can be improved, and the falling off of the second wire can be prevented.

In the embodiments of the present application, the length by which the first microneedle 51 extends from the end side of the operating handle 70 is 1-3 mm, and the length by which the second microneedle 61 extends from the end side of the operating handle 70 is 1-3 mm. In this way, the puncturing effect of the first microneedle 51 and the second microneedle 61 on the target tissue can be ensured, and the energy of the pulses emitted by the first microneedle 51 and the second microneedle 61 to the target tissue can also be ensured, thereby ensuring the therapeutic effect of the oral ablation device on the pulse ablation of the target tissue.

In the embodiments of the present application, the operating handle 70 is a variable-diameter operating handle, and the radial dimension of the variable-diameter operating handle decreases from the proximal end to the distal end.

Specifically, the radial dimension of the variable-diameter operating handle gradually decreases from the proximal end to the distal end. In this way, the gripping comfort and operational convenience of the oral ablation device can be improved.

In the embodiments of the present application, the shape of the first electrode 50 is sheet-like, and the shape of the second electrode 60 is sheet-like.

Specifically, the first electrode 50 can be cut from a metal sheet. Correspondingly, the second electrode 60 can be cut from a metal sheet.

In the embodiments of the present application, both the first electrode 50 and the second electrode 60 are sheet-like electrodes, which facilitate the abutting and fixing of the first electrode 50 and the second electrode 60 respectively to the electrode adapter 72, can improve the fixing effects of the electrode adapter 72 and the electrode fixing part 73 on the first electrode 50 and the second electrode 60 respectively, and thus further prevent the displacement and falling off of the first electrode 50 and the second electrode 60.

In the embodiments of the present application, the oral ablation device further includes a sensing device and a control device. The sensing device is electrically connected to the control device, and the control device is respectively electrically connected to the first electrode 50 and the second electrode 60. The sensing device is used to obtain the electric field environment parameters of the target tissue and send them to the control device. The control device is used to control the first electrode 50 and the second electrode 60 to emit pulses to the target tissue based on the electric field environment parameters. In this way, the operational safety of the oral ablation device can be improved.

Specifically, the electric field environment parameters can include current and voltage.

Specifically, based on the electric field environment parameters, it can be determined whether the target tissue is lesioned tissue. In the case where it is determined that the target tissue is lesioned tissue, the first electrode 50 and the second electrode 60 are controlled to emit pulses to the target tissue.

Further, the sensing device is also used to obtain the abutment parameters between the distal end of the operating handle 70 and the target tissue. In the case where the abutment parameters indicate that the abutment between the distal end of the operating handle 70 and the target tissue is reasonable, the first electrode 50 and the second electrode 60 are controlled to emit pulses to the target tissue. In this way, the operational safety and the therapeutic efficiency of pulse ablation of the oral ablation device can be improved.

In some embodiments, the oral ablation device can be used in combination with a neutral electrode. For example, a neutral electrode can be attached to the target tissue as needed, and the oral ablation device can identify the neutral electrode. In the case where the oral ablation device identifies the neutral electrode, the first electrode 50 and the second electrode 60 are controlled to be of the same polarity and form an electrode pair with the neutral electrode to emit pulses to the target tissue.

Specifically, since the distance between the neutral electrode and the first electrode 50 and the second electrode 60 is farther than the spacing distance between the first electrode 50 and the second electrode 60, the pulse radiation range is relatively large, but the pulse energy is relatively low, which is suitable for target tissues with a large lesion range and a shallow lesion depth.

Correspondingly, when the oral ablation device is used alone, the electrode pair formed by the first electrode 50 and the second electrode 60 emits pulses to the target tissue with a relatively concentrated range and relatively high pulse energy, which is suitable for target tissues with a small lesion range and a deep lesion depth.

The following describes specific embodiments of this application based on the above technical solutions.

### Embodiment 5

Referring to Figures 10-18, Embodiment 1 provides an oral ablation device, comprising a first electrode 50, a second electrode 60, and an operating handle 70. The first electrode 50 is fixedly connected to the operating handle 70, the second electrode 60 is fixedly connected to the operating handle 70, and the first electrode 50 and the second electrode 60 are arranged opposite to each other. The first electrode 50 has at least one first microneedle 51, the second electrode 60 has at least one second microneedle 61, and both the first microneedle 51 and the second microneedle 61 extend from the end side of the operating handle 70. The first electrode 50 and the second electrode 60 are used to emit pulses to the target tissue through the first microneedle 51 and the second microneedle 61.

The first electrode 50 and the second electrode 60 are respectively fixedly connected to the distal end of the operating handle 70, and the operating handle 70 is used to fix the relative positions of the first electrode 50 and the second electrode 60.

The first electrode 50 has multiple first microneedles 51, and the second electrode 60 has multiple second microneedles 61. The arrangement of multiple first microneedles 51 and multiple second microneedles 61 can enhance the energy of pulses emitted by the first electrode 50 and the second electrode 60.

The first microneedle 51 is used to pierce and extend into the target tissue, and correspondingly, the second microneedle 61 is used to pierce and extend into the target tissue. The first electrode 50 and the second electrode 60 form an electrode pair, which emits pulses within the target tissue through the first microneedle 51 and the second microneedle 61 to perform ablation treatment on the target tissue.

Both the first electrode 50 and the second electrode 60 are sheet-like electrodes. The first electrode 50 can be cut from a metal sheet, and correspondingly, the second electrode 60 can be cut from a metal sheet.

The first electrode 50 includes multiple first microneedles 51 and a first electrode connecting portion 54 fixedly connected to the multiple first microneedles 51. The multiple first microneedles 51 are arranged adjacent to each other on the end side of the first electrode connecting portion 54, and a first arc-shaped connecting structure is formed between adjacent first microneedles 51. The second electrode 60 includes multiple second microneedles 61 and a second electrode connecting portion 64 fixedly connected to the multiple second microneedles 61. The multiple second microneedles 61 are arranged adjacent to each other on the end side of the second electrode connecting portion 64, and a second arc-shaped connecting structure is formed between adjacent second microneedles 61.

The multiple first microneedles 51 are arranged adjacent to each other on the distal end side of the first electrode connecting portion 54, and adjacent first microneedles 51 are smoothly connected through the first arc-shaped connecting structure.

The multiple second microneedles 61 are arranged adjacent to each other on the distal end side of the second electrode connecting portion 64, and adjacent second microneedles 61 are smoothly connected through the second arc-shaped connecting structure.

The first electrode 50 includes five first microneedles 51, and correspondingly, the second electrode 60 includes five second microneedles 61.

The cross-sectional dimension at the distal end of the first microneedle 51 is smaller than that at the proximal end of the first microneedle 51. The cross-sectional dimension at the distal end of the second microneedle 61 is smaller than that at the proximal end of the second microneedle 61.

The shape of the first microneedle 51 is conical.

The shape of the second microneedle 61 is conical.

The oral ablation device also includes a first lead and a second lead. The first electrode connecting portion 54 is provided with a first lead connection holeconnection hole 55, which is used for the first lead to pass through and be fixedly connected to the first lead. The second electrode connecting portion 64 is provided with a second lead connection hole 65, which is used for the second lead to pass through and be fixedly connected to the second lead.

The first lead connection hole 55 penetrates through the first electrode 50. The first lead is fixedly connected to the first electrode 50 through the first lead connection hole 55. Specifically, the first lead is welded to the first electrode 50 at the first lead connection hole 55.

The second lead connection hole 65 penetrates through the second electrode 60. The second lead is fixedly connected to the second electrode 60 through the second lead connection hole 65. Specifically, the second lead is welded to the second electrode 60 at the second lead connection hole 65.

The length by which the first microneedle 51 extends from the end side of the operating handle 70 is 1.5 mm, and the length by which the second microneedle 61 extends from the end side of the operating handle 70 is 1.5 mm.

The operating handle 70 includes a support housing 71 and an electrode adapter 72. The electrode adapter 72 is fixedly connected to the end side of the support housing 71. One end of the first electrode 50 opposite to the tip of the first microneedle 51 and one end of the second electrode 60 opposite to the tip of the second microneedle 61 are respectively fixedly connected to the end of the electrode adapter 72 away from the support housing 71. The first electrode 50 and the second electrode 60 are fixed to the opposite end sides of the end of the electrode adapter 72 away from the support housing 71.

The electrode adapter 72 is in pin-hole cooperation with the support housing 71, and the electrode adapter 72 is fixedly connected to the support housing 71 by laser welding.

The proximal end of the first electrode 50 is fixedly connected to the distal end of the electrode adapter 72. Correspondingly, the proximal end of the second electrode 60 is fixedly connected to the distal end of the electrode adapter 72.

The first electrode 50 and the second electrode 60 are arranged on opposite sides of the electrode adapter 72, with a preset spacing distance between the first electrode 50 and the second electrode 60.

The first electrode 50 and the second electrode 60 are fixedly connected to the opposite sides of the electrode adapter 72 at a preset spacing distance.

The operating handle 70 also includes an electrode fixing part 73. The electrode fixing part 73 is sleeved on the electrode adapter 72 and covers the connecting portion between the first electrode 50 and the electrode adapter 72 and the connecting portion between the second electrode 60 and the electrode adapter 72. The electrode fixing part 73 is used to fix the first electrode 50 and the second electrode 60 respectively to the end side of the electrode adapter 72. Both the first microneedle 51 and the second microneedle 61 extend outward from the end side of the electrode fixing part 73.

The electrode adapter 72 and the electrode fixing part 73 cooperate to fix the first electrode 50 and the second electrode 60.

The electrode fixing part 73 can be a soft rubber coating. Through injection molding, the electrode fixing part 73 is wrapped and fixed on the electrode adapter 72 to fix the first electrode 50 and the second electrode 60.

Both the first microneedle 51 and the second microneedle 61 completely extend out of the end side of the electrode fixing part 73.

The support housing 71 includes a first housing 711 and a second housing 712. After the first housing 711 and the second housing 712 are fixedly connected, an inner cavity of the housing is formed. One end of the electrode adapter 72 extends into the inner cavity of the housing and is fixed between the first housing 711 and the second housing 712. At least one of the first housing 711 and the second housing 712 extends into the electrode fixing part 73 and is fixedly connected to the electrode adapter 72.

The first housing 711 and the second housing 712 are in pin-hole cooperation and can be fixedly connected by laser welding.

The proximal end of the electrode adapter 72 can extend into the inner cavity of the housing and be fixedly clamped between the first housing 711 and the second housing 712.

The distal end of the first housing 711 protrudes relative to the distal end of the second housing 712, and the distal end of the first housing 711 is fixedly connected to the electrode adapter 72. Specifically, the electrode fixing part 73 can wrap and fix the distal end of the electrode adapter 72 and the distal end of the first housing 711, and the distal end of the first housing 711 extends into the electrode fixing part 73.

The distal end of the first housing 711 and the electrode adapter 72 are in pin-hole cooperation and can be fixedly connected by laser welding.

The end of the electrode adapter 72 has a first electrode positioning portion 721 and a second electrode positioning portion 722. The first electrode 50 has a third electrode positioning portion 52 that matches the first electrode positioning portion 721. The first electrode positioning portion 721 and the third electrode positioning portion 52 can abut against each other to limit the movement of the first electrode 50. The second electrode 60 has a fourth electrode positioning portion 62 that matches the second electrode positioning portion 722. The second electrode positioning portion 722 and the fourth electrode positioning portion 62 can abut against each other to limit the movement of the second electrode 60.

The first electrode positioning portion 721 includes a first stop portion 3211 and a second stop portion 3212. Correspondingly, the third electrode positioning portion 52 includes a third stop portion 121 that matches the first stop portion 3211 and a fourth stop portion 122 that matches the second stop portion 3212. The cooperation and abutment of the first stop portion 3211 and the third stop portion 121, and the cooperation and abutment of the second stop portion 3212 and the fourth stop portion 122 can limit the movement of the first electrode 50 relative to the electrode adapter 72.

The second electrode positioning portion 722 includes a fifth stop portion 3221 and a sixth stop portion 3222. Correspondingly, the fourth electrode positioning portion 62 includes a seventh stop portion 221 that matches the fifth stop portion 3221 and an eighth stop portion 222 that matches the sixth stop portion 3222. The cooperation and abutment of the fifth stop portion 3221 and the seventh stop portion 221, and the cooperation and abutment of the sixth stop portion 3222 and the eighth stop portion 222 can limit the movement of the second electrode 60 relative to the electrode adapter 72.

The end of the electrode adapter 72 is provided with multiple grooves 723, and the electrode fixing part 73 can extend into the grooves 723.

The end of the electrode adapter 72 also has a first electrode assembly pin 724 and a second electrode assembly pin 725. The first electrode 50 has a first electrode assembly hole 53 that matches the first electrode assembly pin 724, and the first electrode assembly pin 724 can extend through the first electrode assembly hole 53. The second electrode 60 has a second electrode assembly hole 63 that matches the second electrode assembly pin 725, and the second electrode assembly pin 725 can extend through the second electrode assembly hole 63.

The first electrode 50 is sleeved on the first electrode assembly pin 724 through the first electrode assembly hole 53.

The second electrode 60 is sleeved on the second electrode assembly pin 725 through the second electrode assembly hole 63.

The oral ablation device also includes a sensing device and a control device. The sensing device and the control device are electrically connected, and the control device is respectively electrically connected to the first electrode 50 and the second electrode 60. The sensing device is used to obtain the electric field environment parameters of the target tissue and send them to the control device. The control device is used to control the first electrode 50 and the second electrode 60 to emit pulses to the target tissue based on the electric field environment parameters.

The electric field environment parameters can include current and voltage.

Based on the electric field environment parameters, it can be determined whether the target tissue is diseased tissue. In the case of determining that the target tissue is diseased tissue, the first electrode 50 and the second electrode 60 are controlled to emit pulses to the target tissue.

The sensing device is also used to obtain the abutment parameters between the distal end of the operating handle 70 and the target tissue. In the case where the abutment parameters indicate that the distal end of the operating handle 70 abuts reasonably against the target tissue, the first electrode 50 and the second electrode 60 are controlled to emit pulses to the target tissue.

The oral ablation device can be used in combination with a neutral electrode. A neutral electrode can be attached to the target tissue as needed, and the oral ablation device can identify the neutral electrode. In the case where the oral ablation device identifies the neutral electrode, the first electrode 50 and the second electrode 60 are controlled to be of the same polarity, forming an electrode pair with the neutral electrode to emit pulses to the target tissue.

The operating handle 70 includes multiple operating areas connected in sequence, and the operating area includes an arc-shaped section, with the arc-shaped sections of adjacent operating areas bending in different directions.

The multiple operating areas include a first gripping area 74, a second gripping area 75, and an insertion area 76. The first gripping area 74, the second gripping area 75, and the insertion area 76 are connected in sequence in an S-shape, and the insertion area 76 is respectively connected to the first electrode 50 and the second electrode 60.

The arc-shaped section of the first gripping area 74 and the arc-shaped section of the second gripping area 75 bend in different directions, and the arc-shaped section of the second gripping area 75 and the arc-shaped section of the insertion area 76 bend in different directions.

The first gripping area 74 is arranged at the proximal end of the support housing 71, the second gripping area 75 is arranged at the distal end of the support housing 71, and the insertion area 76 is arranged on the electrode fixing part 73.

The oral ablation device has an axial direction of the operating handle. There is a first bending angle between the first gripping area 74 and the axial direction of the operating handle, and the first bending angle is 20°. There is a second bending angle between the second gripping area 75 and the axial direction of the operating handle, and the second bending angle is 20°. There is a third bending angle between the insertion area 76 and the axial direction of the operating handle, and the third bending angle is 45°.

The operating handle 70 is a variable-diameter operating handle, and the radial dimension of the variable-diameter operating handle decreases from the proximal end to the distal end.

### Embodiment 6

The difference between Embodiment Six and Embodiment Five lies in the settings of the first bending angle, the second bending angle, and the third bending angle. The similarities between Embodiment Six and Embodiment Five will not be elaborated on here. The differences between Embodiment Six and Embodiment Five are now described as follows.

The operating handle 70 comprises multiple operating zones connected in sequence. The operating zones include arc-shaped segments, and the arc-shaped segments of adjacent operating zones bend in different directions.

The multiple operating zones include a first gripping area 74, a second gripping area 75, and an insertion area 76. The first gripping area 74, the second gripping area 75, and the insertion area 76 are connected in sequence to form an S-shape, and the insertion area 76 is connected to both the first electrode 50 and the second electrode 60.

The arc-shaped segment of the first gripping area 74 bends in a different direction from that of the arc-shaped segment of the second gripping area 75, and the arc-shaped segment of the second gripping area 75 bends in a different direction from that of the arc-shaped segment of the insertion area 76.

The first gripping area 74 is located at the proximal end of the support housing 71, the second gripping area 75 is located at the distal end of the support housing 71, and the insertion area 76 is arranged on the electrode fixing part 73.

The oral ablation device has an axial direction of the operating handle. There is a first bending angle between the first gripping area 74 and the axial direction of the operating handle, and the first bending angle is 25°. There is a second bending angle between the second gripping area 75 and the axial direction of the operating handle, and the second bending angle is 25°. There is a third bending angle between the insertion area 76 and the axial direction of the operating handle, and the third bending angle is 40°.

The above are merely preferred embodiments of this application and are not intended to limit this application. Any modifications, equivalent substitutions, improvements, etc., made within the spirit and principles of this application shall be included within the protection scope of this application.

## Claims

1. A pulse ablation device for oral lesion tissue, comprising an outer electrode (10), an inner electrode (20), an electrode fixing device (30), and an operating rod (40);
the outer electrode (10) is fixedly connected to the electrode fixing device (30), the inner electrode (20) is fixedly connected to the electrode fixing device (30), and the electrode fixing device (30) is fixedly connected to the operating rod (40);
both the outer electrode (10) and the inner electrode (20) are arranged at a distal end of the electrode fixing device (30);
the outer electrode (10) is arranged around an outer peripheral side of the inner electrode (20), a discharge gap is formed between the outer electrode (10) and the inner electrode (20), and the discharge gap is configured to be broken down by current to form pulses.

2. The pulse ablation device according to claim 1, wherein the electrode fixing device (30) comprises an electrode fixing base (31) and a rubber-coated part (32), and the electrode fixing base (31) is fixedly connected to the operating rod (40);
proximal ends of the outer electrode (10) and the inner electrode (20) are both fixedly connected to a distal end of the electrode fixing base (31);
the rubber-coated part (32) is wrapped around a distal end of the electrode fixing base (31) and is configured to fix the outer electrode (10) and the inner electrode (20) on the electrode fixing base (31).

3. The pulse ablation device according to claim 2, wherein both the outer electrode (10) and the inner electrode (20) protrude from a distal end side of the rubber-coated part (32).

4. The pulse ablation device according to claim 3, wherein a protruding height of the outer electrode (10) from the distal end side of the rubber-coated part (32) is 0.1-0.3 mm, and a protruding height of the inner electrode (20) from the distal end side of the rubber-coated part (32) is 0.1-0.3 mm.

5. The pulse ablation device according to claim 2, wherein the operating rod (40) comprises a first sub-shell (41) and a second sub-shell (42) that are fixedly connected;
a proximal end of the electrode fixing base (31) is fixedly arranged between the first sub-shell (41) and the second sub-shell (42);
a distal end of at least one of the first sub-shell (41) and the second sub-shell (42) is fixedly connected to the electrode fixing base (31) and extends into the rubber-coated part (32).

6. The pulse ablation device according to any one of claims 2-5, wherein an outer electrode positioning structure (311) is arranged on the distal end side of the electrode fixing base (31), and the outer electrode (10) is fixedly connected to the outer electrode positioning structure (311);
the outer electrode positioning structure (311) is provided with an outer electrode abutting surface (3111), and at least one first recessed groove (3112) is recessed on the outer electrode abutting surface (3111).

7. The pulse ablation device according to claim 6, wherein an inner electrode positioning structure (312) is arranged on the distal end side of the electrode fixing base (31), and the inner electrode (20) is fixedly connected to the inner electrode positioning structure (312);
the inner electrode positioning structure (312) is provided with an inner electrode abutting surface (3121), and at least one second recessed groove (3122) is recessed on the inner electrode abutting surface (3121).

8. The pulse ablation device according to claim 7, wherein the outer electrode positioning structure (311) is arranged on an outer peripheral side of the inner electrode positioning structure (312), the outer electrode positioning structure (311) is spaced apart from the inner electrode positioning structure (312), and a first gap (313) is provided between the outer electrode positioning structure (311) and the inner electrode positioning structure (312);
at least one through-hole (314) is arranged on the outer electrode positioning structure (311), and the through-hole (314) is in communication with the first gap (313).

9. The pulse ablation device according to any one of claims 2-5, wherein the pulse ablation device further comprises an outer electrode connecting wire and an inner electrode connecting wire;
the outer electrode connecting wire is fixedly connected to the outer electrode (10), and the inner electrode connecting wire is fixedly connected to the inner electrode (20);
at least one wire retaining slot (315) is arranged on the electrode fixing base (31), and the outer electrode connecting wire and the inner electrode connecting wire are respectively engaged with the wire retaining slot (315).

10. The pulse ablation device according to claim 9, wherein a first wire routing hole (316) is arranged on the electrode fixing base (31), a second wire routing hole (43) is arranged inside the operating rod (40), the first wire routing hole (316) is in communication with the second wire routing hole (43), and the wire retaining slot (315) is arranged on the first wire routing hole (316);
both the first wire routing hole (316) and the second wire routing hole (43) are configured to accommodate the outer electrode connecting wire and the inner electrode connecting wire.

11. An oral ablation device, comprising a first electrode (50), a second electrode (60), and an operating handle (70);
the first electrode (50) is fixedly connected to the operating handle (70), the second electrode (60) is fixedly connected to the operating handle (70), and the first electrode (50) and the second electrode (60) are arranged opposite to each other;
the first electrode (50) is provided with at least one first microneedle (51), the second electrode (60) is provided with at least one second microneedle (61), and both the first microneedle (51) and the second microneedle (61) extend from one end side of the operating handle (70);
the first electrode (50) and the second electrode (60) are configured to emit pulses to the target tissue through the first microneedle (51) and the second microneedle (61).

12. The oral ablation device according to claim 11, wherein the operating handle (70) comprises a support housing (71) and an electrode adapter (72);
the electrode adapter (72) is fixedly connected to one end side of the support housing (71);
one end of the first electrode (50) opposite to the needle tip of the first microneedle (51) and one end of the second electrode (60) opposite to the needle tip of the second microneedle (61) are respectively fixedly connected to the end of the electrode adapter (72) away from the support housing (71), and the first electrode (50) and the second electrode (60) are fixed on the opposite end sides of the end of the electrode adapter (72) away from the support housing (71).

13. The oral ablation device according to claim 12, wherein the operating handle (70) further comprises an electrode fixing part (73);
the electrode fixing part (73) is sleeved on the electrode adapter (72) and covers a connecting portion, between the first electrode (50) and the electrode adapter (72), and a connecting portion, between the second electrode (60) and the electrode adapter (72), and the electrode fixing part (73) is configured to respectively fix the first electrode (50) and the second electrode (60) on the end sides of the electrode adapter (72);
both the first microneedle (51) and the second microneedle (61) extend outward from the end sides of the electrode fixing part (73).

14. The oral ablation device according to claim 13, wherein the support housing (71) comprises a first shell (711) and a second housing (712), and a shell cavity is formed after the first shell (711) and the second housing (712) are fixedly connected;
one end of the electrode adapter (72) extends into the shell cavity and is fixed between the first shell (711) and the second housing (712);
at least one of the first shell (711) and the second housing (712) extends into the electrode fixing part (73) and is fixedly connected to the electrode adapter (72).

15. The oral ablation device according to claim 12, wherein the end of the electrode adapter (72) is provided with a first electrode positioning portion (721) and a second electrode positioning portion (722);
the first electrode (50) is provided with a third electrode positioning portion (52) adapted to the first electrode positioning portion (721), and the first electrode positioning portion (721) abuts against the third electrode positioning portion (52) to limit movement of the first electrode (50);
the second electrode (60) is provided with a fourth electrode positioning portion (62) adapted to the second electrode positioning portion (722), and the second electrode positioning portion (722) abuts against the fourth electrode positioning portion (62) to limit movement of the second electrode (60).

16. The oral ablation device according to claim 12, wherein a groove (723) is arranged at one end of the electrode adapter (72), and the electrode fixing part (73) extends into the groove (723).

17. The oral ablation device according to claim 15, wherein one end of the electrode adapter (72) further comprises a first electrode assembly pin (724) and a second electrode assembly pin (725);
the first electrode (50) is provided with a first electrode assembly hole (53) adapted to the first electrode assembly pin (724), and the first electrode assembly pin (724) extends out from the first electrode assembly hole (53);
the second electrode (60) is provided with a second electrode assembly hole (63) adapted to the second electrode assembly pin (725), and the second electrode assembly pin (725) extends out from the second electrode assembly hole (63).

18. The oral ablation device according to any one of claims 11-17, wherein the first electrode (50) comprises multiple first microneedles (51) and a first electrode connecting portion (54) fixedly connected to the multiple first microneedles (51), the multiple first microneedles (51) are arranged adjacent to each other on the end side of the first electrode connecting portion (54), and a first arc-shaped connecting structure is formed between adjacent first microneedles (51);
the second electrode (60) comprises multiple second microneedles (61) and a second electrode connecting portion (64) fixedly connected to the multiple second microneedles (61), the multiple second microneedles (61) are arranged adjacent to each other on the end side of the second electrode connecting portion (64), and a second arc-shaped connecting structure is formed between adjacent second microneedles (61).

19. The oral ablation device according to any one of claims 11-17, wherein a cross-sectional size of a distal end of the first microneedle (51) is smaller than that of a proximal end of the first microneedle (51);
a cross-sectional size of a distal end of the second microneedle (61) is smaller than that of a proximal end of the second microneedle (61).

20. The oral ablation device according to any one of claims 11-17, wherein the first microneedle (51) comprises a first tapered end, and the first tapered end is arranged at a distal end of the first microneedle (51);
the second microneedle (61) comprises a second tapered end, and the second tapered end is arranged at a distal end of the second microneedle (61).
